# EUROPEAN PATENT SPECIFICATION

(11) **EP 3 810 074 B1**
(45) Date of publication and mention of the grant of the patent: **07.10.2026**
(21) Application number: 19732045.0
(22) Date of filing: 24.06.2019
(51) Int. Cl.: A61K 8/37, A61K 8/34, A61Q 5/02, A61K 8/92, A61K 8/73, A61K 8/41

(54) **COMPOSITION COMPRISING A MONOESTER OF A FATTY ACID AND OF POLYGLYCEROL, AN OIL, A POLYOL, AND A CATIONIC AGENT**
ZUSAMMENSETZUNG MIT EINEM MONOESTER EINER FETTSÄURE UND VON POLYGLYCERIN, EINEM ÖL, EINEM POLYOL UND EINEM KATIONISCHEN AGENS
COMPOSITION COMPRENANT UN MONOESTER D'UN ACIDE GRAS ET DE POLYGLYCÉROL, UNE HUILE, UN POLYOL ET UN AGENT CATIONIQUE

(30) Priority: 25.06.2018 FR 1855659
(43) Date of publication of application: 28.04.2021
(73) Proprietor: L'OREAL, 75008 Paris (FR)
(72) Inventor: BENAZZOUZ, Adrien, 93400 SAINT OUEN (FR); MALOUG, Saber, 93400 SAINT OUEN (FR); MATHONNEAU, Estelle, 93400 Saint-Ouen (FR); HOPP, Florian, 94152 CHEVILLY-LARUE (FR)
(74) Representative: Casalonga
(86) International application number: PCT/EP2019/066689
(87) International publication number: WO 2020/002248

(56) References cited:
- WO-A1-2014/098266
- WO-A1-2017/182401
- FR-A1- 2 855 721
- FR-A1- 3 015 246
- JP-A- 2003 327 506
- T V DROVETSKAYA ET AL: "New high-charge density hydrophobically modified cationic HEC polymers for improved co-deposition of benefit agents and serious conditioning for problem hair", JOURNAL OF COSMETIC SCIENCE, 31 August 2007 (2007-08-31), pages 421 - 434, XP055235466, Retrieved from the Internet <URL:http://journal.scconline.org//pdf/cc2007/cc058n04/p00421-p00434.pdf> [retrieved on 20151210]
- PATEL C U: "ANTI-STATIC PROPERTIES OF SOME CATIONIC POLYMERS USED IN HAIR CARE PRODUCTS PROPRIETES ANTI-STATIQUES DE CERTAINS POLYMERES CATIONIQUES UTILISES DANS LES PRODUITS DE SOINS POUR LES CHEVEUX", INTERNATIONAL JOURNAL OF COSMETIC SCIE, KLUWER ACADEMIC PUBLISHERS, DORDRECHT, NL, vol. 5, no. 5, 1 January 1983 (1983-01-01), pages 181 - 188, XP008021180, ISSN: 0142-5463
- JURCZYK MATTHEW F ET AL: "CATIONIC SURFACTANTS AND QUATERNARY DERIVATIVES FOR HAIR AND SKIN CARE", COSMETIC SCIENCE AND TECHNOLOGY SERIES, DEKKER, NEW YORK, NY, US, vol. 21, 1 January 1999 (1999-01-01), pages 223 - 249, XP001249398, ISSN: 0887-6541

## Description

The present invention relates to a composition comprising one or more monoesters of a fatty acid and of (poly)glycerol in a content of greater than 10% by weight, one or more liquid fatty substances, one or more polyols including at least one diol, one or more cationic agents, and water.

The invention also relates to a process for washing and/or caring for human keratin fibres, comprising at least one step of applying a composition according to the invention to said human keratin fibres.

It is common practice to use detergent aqueous compositions (such as shampoos) based essentially on anionic surfactants, for cleansing and/or washing keratin fibres such as the hair. However, these compositions are not always entirely satisfactory in terms of the care/conditioning provided to the keratin fibres.

In parallel, oil-based compositions are often used in hair cosmetics for their beneficial properties in terms of keratin fibre care. However, these oil-based compositions have a tendency to leave a feel perceived to be greasy, charged and insufficiently clean by users. They are generally not satisfactory for hair cleansing.

In particular, FR 3 015 246 A1 discloses a cosmetic emulsion comprising at least one oil, at least one non-ionic surfactant and at least one glycoside compound. The application WO 2014/098266 A1also discloses a cosmetic emulsion comprising at least one oil, at least two (poly)glyceryl fatty acid ester surfactants, at least one polyol and water. The application WO 2017/182401 A1 discloses a cosmetic composition comprising anionic, amphoteric and non-ionic surfactants, at least one polyol and at least one cationic and/or amphoteric polymers.

Moreover, the users of cosmetic products can be put off by the not very aesthetic appearance of certain opaque compositions for cleansing or washing keratin fibres. These users are in particular searching for compositions which are more aesthetic, fluid or even transparent.

There is therefore a real need for aesthetic compositions which make it possible to obtain excellent cosmetic properties, in particular in terms of disentangling, suppleness, manageability, softness to the touch and straightening of treated hair, while same time having good properties of use *(i.e.* ease of application) and good detergent properties, and in particular a good intrinsic washing power.

These objectives are achieved with the present invention, a subject of which is in particular a cosmetic composition comprising:
i) one or more monoesters of a fatty acid and of (poly)glycerol in a total content of greater than 10% by weight relative to the total weight of the composition,
ii) one or more fatty substances, liquid at 30°C and at 1.013×10⁵ Pa, chosen from hydrocarbons, fatty alcohols comprising from 8 to 40 carbon atoms, fatty esters comprising from 8 to 40 carbon atoms, fatty ethers comprising from 8 to 40 carbon atoms, silicones and mixtures thereof,
iii) one or more polyols including at least one diol,
iv) one or more cationic agents chosen from cationic polymers and/or cationic surfactants; and
v) water, in a content ranging from 1% to 20% by weight relative to the total weight of the composition;
it being understood that the weight ratio of, on the one hand, the total content of monoester(s) of a fatty acid and of (poly)glycerol to, on the other hand, the total content of diol(s) is greater than or equal to 1.5, preferably greater than or equal to 2, better still greater than or equal to 2.5, or even greater than or equal to 3.

The composition according to the invention has a good detergent power.

It has also been noted that hair treated with the composition according to the invention is particularly clean, light and uncharged to the touch.

The composition according to the invention also has very good conditioning properties.

It has thus been noted that the composition according to the invention gives the hair a particularly soft and smooth feel, and also gives the hair sheen and suppleness.

In addition, the composition according to the invention has good properties of use, especially a good ease of application, which leads to a better spread of the composition on the hair. The composition according to the invention further reduces the static electricity of the hair (fly away effect).

Furthermore, it has been observed that hair treated with the composition according to the invention is easier to disentangle and more manageable.

The composition according to the invention is advantageously clear, which gives it a particularly attractive aesthetic appearance that is highly sought after by users.

A subject of the invention is also a process for for washing and/or caring for human keratin fibres such as the hair, comprising at least one step of applying, to said human keratin fibres, a composition according to the invention.

A subject of the invention is also the use of the composition according to the invention for washing and/or caring for human keratin fibres such as the hair.

Other subjects, characteristics, aspects and advantages of the invention will emerge even more clearly on reading the description and the example that follows.

In the present description, and unless otherwise indicated:
- the expression "at least one" is equivalent to the expression "one or more" and can be replaced therewith;
- the expression "between" is equivalent to the expression "ranging from" and can be replaced therewith, and implies that the limits are included;
- for the purposes of the present invention, the expression "greater than" and respectively the expression "less than" are intended to mean an open range which is strictly greater, respectively strictly less, and therefore that the limits are not included;
- according to the present application, the term "(poly)glycerol" denotes a compound comprising in its structure one or more mol of glycerol;
- according to the present application, the term "polyglycerol" denotes a compound comprising in its structure at least two mol of glycerol.

Advantageously, the composition according to the present invention is seemingly single-phase. For the purposes of the present invention, the expression "seemingly single-phase" is intended to mean that the composition according to the invention comprises, at ambient temperature (25°C) and atmospheric pressure, two or more phases, one of the phases being dispersed in the other, such that the phases cannot be distinguished from one another with the naked eye.

According to one preferred embodiment of the invention, the composition is in the form of a microemulsion.

The term "microemulsion" denotes, in a manner known per se, not a true emulsion, but a thermodynamically stable, clear solution of swollen micelles. The term microemulsion is in particular defined in the "Dictionary of Colloid and Surface Science" by Paul Becher, published in 1990 by Marcel Dekker Inc, page 102.

More preferentially, the composition is in the form of an oil-in-water microemulsion, having a volume-average size of oil drops of less than or equal to 10 µm, even more preferentially less than 5 µm, better still ranging from 0.01 to 1 µm, even better still ranging from 0.05 to 0.5 µm, and even ranging from 0.1 to 0.4 µm.

The volume-average size of the particles (or oil drops) may be determined in particular according to the known method of dynamic light scattering (DLS). By way of apparatus that can be used for this determination, mention may be made of the particle size analyser of the brand Malvern, model Zetasizer Nano ZS, equipped with a standard laser having a power of 4 mW, and at a wavelength of 633 nm. This device is also equipped with a correlator (25 ns to 8000 s, 4000 channels max.).

These microemulsions differ from nanoemulsions, which are thermodynamically unstable, fine emulsions, which generally require a considerable input of energy in order to prepare them, and which are capable of changing over time.

The composition according to the invention is advantageously in the form of a clear to translucent, more preferentially clear, fluid.

The clarity of the composition according to the invention can be characterized by the measurement of its turbidity, by turbidemitry (in NTU units). In the context of the present invention, the turbidity measurements were carried out with a turbidimeter, model HI 88713-ISO from the company Hanna Instruments.

Preferably, the turbidity of the compositions according to the invention, measured at ambient temperature (25°C) and atmospheric pressure, is less than 400 NTU units, more preferentially between 1 and 250 NTU units, even better still between 3 and 200 NTU units.

### Monoesters of a fatty acid and of (poly)glycerol

The composition according to the present invention comprises one or more monoesters of a fatty acid and of (poly)glycerol in a total content of greater than 10% by weight relative to the total weight of the composition.

The term "fatty acid" is intended to mean an organic acid comprising in its structure a linear or branched, saturated or unsaturated hydrocarbon-based chain comprising from 8 to 40 carbon atoms, preferably from 10 to 30 carbon atoms, more preferentially from 12 to 22 carbon atoms,

Preferably, the monoester(s) of a fatty acid and of (poly)glycerol comprise on average from 1 to 30 mol of glycerol; more preferentially from 2 to 10 mol of glycerol, better still from 3 to 8 mol of glycerol.

For the purposes of the present invention, the term "average" is intended to mean a number-average.

According to one preferred embodiment of the invention, the monoester(s) of a fatty acid and of (poly)glycerol are chosen from the compounds of formula (I) below: 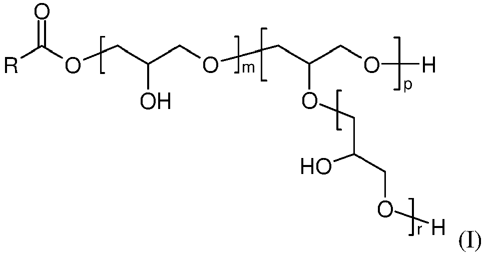 in which:
R represents a linear or branched, saturated or unsaturated C₇-C₃₉, preferably C₉-C₂₉, better still C₁₁-C₂₁, hydrocarbon-based chain;
m, p and r represent an integer between 0 and 30, and are such that the sum of the integers m, p and r is between 1 and 30, better still between 2 and 10, and even better still between 3 and 8.

Preferably, p=r=0, and m is an integer between 1 and 30, more preferentially m is an integer between 2 and 10, or even between 3 and 8.

Preferably, the monoester(s) of a fatty acid and of (poly)glycerol are chosen from monoesters of C₈-C₃₀ fatty acids and of (poly)glycerol; more preferentially from monoesters of C₈-C₃₀ fatty acids and of polyglycerol; even more preferentially from monoesters of oleic acid and of polyglycerol, monoesters of lauric acid and of polyglycerol, and mixtures thereof; even better still from tetraglyceryl monooleate (INCI name: polyglyceryl-4 oleate), pentaglyceryl monooleate (INCI name: polyglyceryl-5 oleate), tetraglyceryl monolaurate (INCI name: polyglyceryl-4 laurate), and mixtures thereof.

Preferably, the total content of monoester(s) of a fatty acid and of (poly)glyceryl present in the composition according to the invention is between 10% and 40% by weight, more preferentially between 12% and 35% by weight, even more preferentially between 15% and 30%, even better still between 20% and 30% by weight, relative to the total weight of the composition.

### Liquid fatty substances

The composition according to the present invention comprises one or more fatty substances, liquid at 30°C and at 1.013×10⁵ Pa, chosen from hydrocarbons, fatty alcohols comprising from 8 to 40 carbon atoms, fatty esters comprising from 8 to 40 carbon atoms, fatty ethers comprising from 8 to 40 carbon atoms, silicones and mixtures thereof.

For the purposes of the present invention, the term "fatty substance" is intended to mean an organic compound that is insoluble in water at 30°C and at atmospheric pressure (760 mmHg, i.e. 1.013×10⁵ Pa), that is to say it has a solubility of less than 5% and preferably less than 1%, even more preferentially less than 0.1%.

The liquid fatty substances are generally soluble in organic solvents under the same temperature and pressure conditions, for instance chloroform, ethanol, benzene, liquid petroleum jelly or decamethylcyclopentasiloxane.

For the purposes of the present invention, the term "liquid fatty substances" is intended to mean a fatty substance which is liquid at 30°C and at atmospheric pressure (760 mmHg, i.e. 1.013×10⁵ Pa).

They preferably have a viscosity of less than or equal to 2 Pa.s, better still less than or equal to 1 Pa.s, and even better still less than or equal to 0.1 Pa.s at a temperature of 25°C and at a shear rate of 1 s⁻¹.

The liquid fatty substances that may be used in the composition according to the invention are generally not oxyalkylenated and preferably do not contain any carboxylic acid COOH functions.

The liquid fatty substances according to the invention are chosen from hydrocarbons, fatty alcohols comprising from 8 to 40 carbon atoms, fatty esters comprising from 8 to 40 carbon atoms, fatty ethers comprising from 8 to 40 carbon atoms, silicones and mixtures thereof.

The term "liquid hydrocarbon" is intended to mean a hydrocarbon composed solely of carbon and hydrogen atoms, which is liquid at a temperature of 30°C and at atmospheric pressure (760 mmHg, i.e. 1.013×10⁵ Pa), of mineral, plant or synthetic origin.

More particularly, the liquid hydrocarbons are chosen from:
- linear or branched, optionally cyclic, C₆-C₁₆ alkanes. Examples that may be mentioned include hexane, undecane, dodecane, tridecane, and isoparaffins, for instance isohexadecane, isododecane and isodecane, and mixtures thereof.
- linear or branched hydrocarbons of mineral, animal or synthetic origin with more than 16 carbon atoms, such as paraffin oils, liquid petroleum jelly, polydecenes, hydrogenated polyisobutene such as the product sold under the brand name Parleam^{®} by NOF Corporation, and squalane.

In one preferred variant, the liquid hydrocarbon(s) are chosen from linear or branched, optionally cyclic, C₆-C₁₆ alkanes.

The term "liquid fatty alcohol" is intended to mean a non-glycerolated and non-oxyalkylenated fatty alcohol, which is liquid at 30°C and at atmospheric pressure (760 mmHg, i.e. 1.013×10⁵ Pa).

Preferably, the liquid fatty alcohols of the invention comprise from 8 to 30 carbon atoms and better still from 8 to 20 carbon atoms.

The liquid fatty alcohols of the invention may be saturated or unsaturated.

The saturated liquid fatty alcohols are preferably branched. They may optionally comprise in their structure at least one aromatic or non-aromatic ring. Preferably, they are acyclic.

More particularly, the saturated liquid fatty alcohols of the invention are chosen from octyldodecanol, isostearyl alcohol and 2-hexyldecanol.

Octyldodecanol is most particularly preferred.

The unsaturated liquid fatty alcohols contain in their structure at least one double or triple bond, and preferably one or more double bonds. When several double bonds are present, there are preferably 2 or 3 of them, and they may be conjugated or unconjugated.

These unsaturated fatty alcohols may be linear or branched.

They may optionally comprise in their structure at least one aromatic or non-aromatic ring. Preferably, they are acyclic.

More particularly, the unsaturated liquid fatty alcohols of the invention are chosen from oleyl alcohol, linoleyl alcohol, linolenyl alcohol and undecylenyl alcohol.

Oleyl alcohol is most particularly preferred.

The term "liquid fatty ester" is intended to mean an ester derived from a fatty acid and/or from a fatty alcohol, which is liquid at 30°C and at atmospheric pressure (760 mmHg, i.e. 1.013×10⁵ Pa) and which is different from the monoester(s) of a fatty acid and of (poly)glyceryl.

The liquid fatty esters are preferably liquid esters of saturated or unsaturated, linear or branched C₁-C₂₆ aliphatic mono- or polyacids and of saturated or unsaturated, linear or branched C₁-C₂₆ aliphatic mono- or polyalcohols, the total number of carbon atoms in the the liquid esters being greater than or equal to 10.

Preferably, for the fatty esters of monoalcohols, at least one from among the alcohol and the acid from which the esters of the invention are derived is branched.

Among the monoesters of monoacids and of monoalcohols, mention may be made of ethyl palmitate, isopropyl palmitate, alkyl myristates such as isopropyl myristate or ethyl myristate, isocetyl stearate, ethyl laurate, 2-ethylhexyl isononanoate, isononyl isononanoate, ethyl octanoate, ethyl caprate, isodecyl neopentanoate and isostearyl neopentanoate.

Esters of C₄-C₂₂ dicarboxylic or tricarboxylic acids and of C₁-C₂₂ alcohols and esters of monocarboxylic, dicarboxylic or tricarboxylic acids and of C₄-C₂₆ dihydroxy, trihydroxy, tetrahydroxy or pentahydroxy non-sugar alcohols may also be used.

Mention may be made especially of diethyl sebacate, diisopropyl sebacate, bis(2-ethylhexyl) sebacate, diisopropyl adipate, di-n-propyl adipate, dioctyl adipate, bis(2-ethylhexyl) adipate, diisostearyl adipate, bis(2-ethylhexyl) maleate, triisopropyl citrate, triisocetyl citrate, triisostearyl citrate, glyceryl trilactate, glyceryl trioctanoate, trioctyldodecyl citrate, trioleyl citrate, neopentyl glycol diheptanoate, and diethylene glycol diisononanoate.

The composition may also comprise, as liquid fatty ester, sugar esters and diesters of C₆-C₃₀ and preferably C₁₂-C₂₂ fatty acids. It is recalled that the term "sugar" is intended to mean oxygen-bearing hydrocarbon-based compounds containing several alcohol functions, with or without aldehyde or ketone functions, and which comprise at least 4 carbon atoms. These sugars may be monosaccharides, oligosaccharides or polysaccharides.

Examples of suitable sugars that may be mentioned include saccharose, glucose, galactose, ribose, fucose, maltose, fructose, mannose, arabinose, xylose and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may comprise one to three conjugated or unconjugated carbon-carbon double bonds.

The esters according to this variant may also be chosen from mono-, di-, tri- and tetraesters, and polyesters, and mixtures thereof.

These esters may be, for example, oleates, laurates, palmitates, myristates, behenates, cocoates, stearates, linoleates, linolenates, caprates and arachidonates, and mixtures thereof, such as, especially, oleopalmitate, oleostearate or palmitostearate mixed esters.

More particularly, use is made of monoesters and diesters and especially of saccharose, glucose or methylglucose mono- or dioleates, stearates, behenates, oleopalmitates, linoleates, linolenates or oleostearates.

An example that may be mentioned is the product sold under the name Glucate^{®} DO by the company Amerchol, which is a methylglucose dioleate.

Finally, use may also be made of natural or synthetic esters of di- or triacids with glycerol.

Among these, mention may be made of plant oils or oils of plant origin.

By way of oils of plant origin or synthetic triglycerides that can be used in the composition of the invention as liquid fatty esters, mention may be made for example of triglyceride oils of plant or synthetic origin, such as liquid fatty acid triglycerides containing from 6 to 30 carbon atoms, for instance heptanoic or octanoic acid triglycerides, or alternatively, for example, sunflower oil, maize oil, soya bean oil, marrow oil, grapeseed oil, sesame oil, hazelnut oil, apricot oil, macadamia oil, arara oil, castor oil, avocado oil, olive oil, rapeseed oil, copra oil, wheatgerm oil, sweet almond oil, apricot oil, safflower oil, candlenut oil, camelina oil, tamanu oil, babassu oil and pracaxi oil, caprylic/capric acid triglycerides, for instance those sold by the company Stéarineries Dubois or those sold under the names Miglyol^{®} 810, 812 and 818 by the company Dynamit Nobel, jojoba oil and shea butter oil.

Preferably, use will be made, as esters according to the invention, of liquid fatty esters derived from mono alcohols, in particular isopropyl myristate or palmitate, or natural or synthetic esters of di- or triacids with glycerol, in particular plant oils.

The liquid fatty ethers may be chosen from liquid dialkyl ethers such as dicaprylyl ether.

The oil(s) that can be used in the composition according to the invention may also be chosen from silicones.

Preferably, the liquid silicone(s) are chosen from polydialkylsiloxanes, especially polydimethylsiloxanes (PDMS), and organomodified polysiloxanes including at least one functional group chosen from amino groups, aryl groups and alkoxy groups.

Organopolysiloxanes are defined in greater detail in Walter Noll's Chemistry and Technology of Silicones (1968), Academic Press. They may be volatile or non-volatile.

The liquid non-volatile silicones that may be used in the composition according to the invention may preferably be liquid non-volatile polydialkylsiloxanes, polyorganosiloxanes modified with organic functional groups chosen from amine groups, aryl groups and alkoxy groups, and also mixtures thereof.

These silicones are more particularly chosen from polydialkylsiloxanes, among which mention may be made mainly of polydimethylsiloxanes bearing trimethylsilyl end groups. The viscosity of the silicones is measured at 25°C according to ASTM standard 445 Appendix C.

Among these polydialkylsiloxanes, mention may be made, in a non-limiting manner, of the following commercial products:
- the Silbione^{®} oils of the 47 and 70 047 series or the Mirasil^{®} oils sold by Rhodia;
- the oils of the Mirasil^{®} series sold by the company Rhodia;
- the oils of the 200 series from Dow Corning;
- the Viscasil^{®} oils from General Electric and certain oils of the SF series (SF 96, SF 18) from General Electric.

Mention may also be made of polydimethylsiloxanes containing dimethylsilanol end groups, known under the name dimethiconol (CTFA), such as the oils of the 48 series from Rhodia.

The organomodified silicones that may be used in accordance with the invention are silicones as defined above and comprising in their structure one or more organofunctional groups attached via a hydrocarbon-based group.

The organomodified silicones may be polydiarylsiloxanes, especially polydiphenylsiloxanes, and polyalkylarylsiloxanes functionalized with the organofunctional groups mentioned previously.

The polyalkylarylsiloxanes are particularly chosen from polydimethyl/methylphenylsiloxanes and polydimethyl/diphenylsiloxanes that are linear and/or branched and that have a viscosity ranging from 1×10⁻⁵ to 5×10⁻² m²/s at 25°C.

Among these polyalkylarylsiloxanes, examples that may be mentioned include the products sold under the following names:
- the Silbione^{®} oils of the 70 641 series from Rhodia;
- oils of the Rhodorsil^{®} 70 633 and 763 series from

Rhodia;
- the oil Dow Corning 556 Cosmetic Grade Fluid from Dow Corning;
- the silicones of the PK series from Bayer, such as the product PK20;
- the silicones of the PN and PH series from Bayer, such as the products PN1000 and PH1000;
- certain oils of the SF series from General Electric, such as SF 1023, SF 1154, SF 1250 and SF 1265.

Among the organomodified silicones, mention may also be made of polyorganosiloxanes comprising:
- substituted or unsubstituted amine groups, for instance the products sold under the names GP 4 Silicone Fluid and GP 7100 by Genesee. The substituted amine groups are in particular C₁-C₄ aminoalkyl groups;
- alkoxylated groups, such as the product sold under the name Silicone Copolymer F-755 by SWS Silicones and Abil Wax^{®} 2428, 2434 and 2440 by the company Goldschmidt.

The volatile silicones are more particularly chosen from those with a boiling point of between 60°C and 260°C, and even more particularly from:
- cyclic silicones comprising from 3 to 7 and preferably 4 to 6 silicon atoms.

These are, for example, octamethylcyclotetrasiloxane sold especially under the name Volatile Silicone 7207 by the company Union Carbide or Silbione 70045 V 2 by the company Rhodia, decamethylcyclopentasiloxane sold under the name Volatile Silicone 7158 by the company Union Carbide, and Silbione 70045 V 5 by the company Rhodia, and mixtures thereof.

Mention may also be made of cyclocopolymers of the dimethylsiloxane/methylalkylsiloxane type, such as Volatile Silicone FZ 3109 sold by the company Union Carbide, of chemical structure:

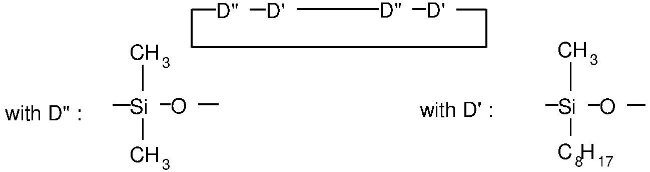

Mention may also be made of mixtures of cyclic silicones with organosilicon compounds, such as the mixture of octamethylcyclotetrasiloxane and tetrakis(trimethylsilyl)pentaerythritol (50/50) and the mixture of octamethylcyclotetrasiloxane and oxy-1,1'-bis(2,2,2',2',3,3'-hexatrimethylsilyloxy)neopentane;
- linear volatile silicones containing 2 to 9 silicon atoms and having a viscosity of less than or equal to 5×10⁻⁶ m²/s at 25°C. Examples include hexamethyldisiloxane, octamethyltrisiloxane and decamethyltetrasiloxane sold especially under the name SH 200 by Toray Silicone. Silicones belonging to this category are also described in the article published in Cosmetics and Toiletries, Vol. 91, Jan. 76, pp. 27-32, Todd & Byers "Volatile Silicone Fluids for Cosmetics". Preferably, the linear volatile silicones contain from 2 to 7 silicon atoms and better still from 3 to 6 silicon atoms.

Preferably, the volatile silicones are chosen from cyclic silicones comprising from 4 to 6 silicon atoms and linear silicones containing 4 to 6 silicon atoms.

According to one preferred embodiment of the invention, the silicones are chosen from amino silicones.

The term "amino silicone" denotes any silicone comprising at least one primary, secondary or tertiary amine or a quaternary ammonium group.

The weight-average molecular masses of these amino silicones may be measured by gel permeation chromatography (GPC) at ambient temperature (25°C), as polystyrene equivalent. The columns used are µ styragel columns. The eluent is THF and the flow rate is 1 ml/min. 200 µl of a 0.5% by weight solution of silicone in THF are injected. Detection is performed by refractometry and UV-metry.

Preferably, the amino silicone(s) that may be used in the context of the invention are chosen from:
a) the polysiloxanes corresponding to formula (A): 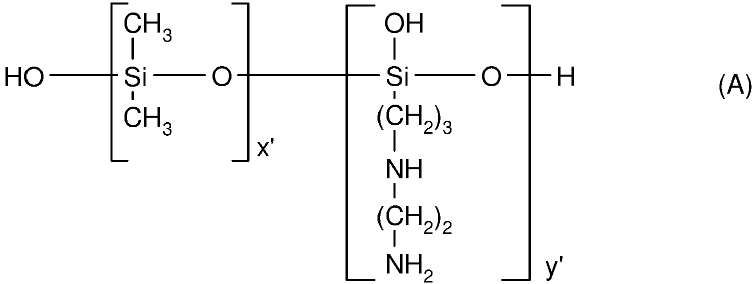 in which x' and y' are integers such that the weight-average molecular weight (Mw) is between 5000 and 500 000 approximately;
b) the amino silicones corresponding to formula (B):

   R'ₐG₃₋ₐ-Si(OSiG₂)ₙ-(OSiG_{b}R'_{2-b})ₘ-O-SiG₃₋ₐ-R'ₐ (B)

   in which:
   - G, which may be identical or different, denotes a hydrogen atom or a phenyl, OH, C₁-C₈ alkyl, for example methyl, or C₁-C₈ alkoxy, for example methoxy, group,
   - a, which may be identical or different, denotes 0 or an integer from 1 to 3, in particular 0,
   - b denotes 0 or 1, in particular 1,
   - m and n are numbers such that the sum (n + m) ranges from 1 to 2000 and in particular from 50 to 150, n possibly denoting a number from 0 to 1999 and especially from 49 to 149, and m possibly denoting a number from 1 to 2000 and especially from 1 to 10;
   - R', which may be identical or different, denotes a monovalent radical of formula -CqH2qL in which q is a number ranging from 2 to 8 and L is an optionally quaternized amine group chosen from the following groups:
      -N(R")₂; -N⁺(R")₃ A-; -NR"-Q-N(R")₂ and -NR"-Q-N⁺(R")₃ A-,
      in which R", which may be identical or different, denotes hydrogen, phenyl, benzyl, or a saturated monovalent hydrocarbon-based radical, for example a C1-C20 alkyl radical; Q denotes a linear or branched group of formula CᵣH₂ᵣ, r being an integer ranging from 2 to 6, preferably from 2 to 4; and A- represents a cosmetically acceptable anion, in particular a halide such as fluoride, chloride, bromide or iodide.

Preferably, the amino silicones are chosen from the amino silicones of formula (B). Preferably, the amino silicones of formula (B) are chosen from the amino silicones corresponding to formulae (C), (D), (E), (F) and/or (G) below.

According to a first embodiment, the amino silicones corresponding to formula (B) are chosen from the silicones known as "trimethylsilyl amodimethicone" corresponding to formula (C): 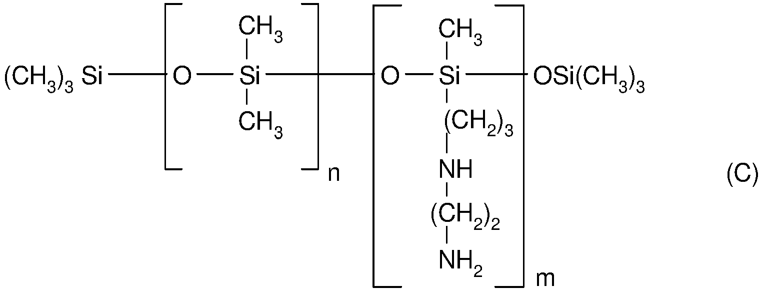 in which m and n are numbers such that the sum (n + m) ranges from 1 to 2000 and in particular from 50 to 150, it being possible for n to denote a number from 0 to 1999 and especially from 49 to 149, and for m to denote a number from 1 to 2000 and in particular from 1 to 10.

According to a second embodiment, the amino silicones corresponding to formula (B) are chosen from the silicones of formula (D) below: 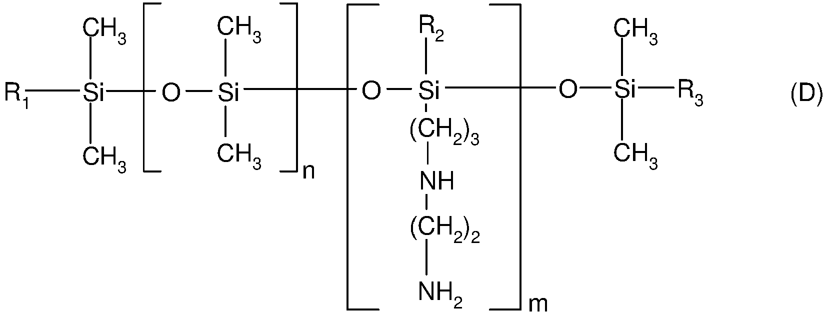 in which:
- m and n are numbers such that the sum (n + m) ranges from 1 to 1000 and in particular from 50 to 250 and more particularly from 100 to 200; it being possible for n to denote a number from 0 to 999 and especially from 49 to 249 and more particularly from 125 to 175, and for m to denote a number from 1 to 1000 and especially from 1 to 10, and more particularly from 1 to 5;
- R1, R2 and R3, which may be identical or different, represent a hydroxyl or C₁-C₄ alkoxy radical, at least one of the radicals R1 to R3 denoting an alkoxy radical.

Preferably, the alkoxy radical is a methoxy radical.

The hydroxy/alkoxy mole ratio preferably ranges from 0.2:1 to 0.4:1 and preferably from 0.25:1 to 0.35:1 and more particularly equals 0.3:1.

The weight-average molecular mass (Mw) of these silicones preferably ranges from 2000 to 1 000 000 and more particularly from 3500 to 200 000.

According to a third embodiment, the amino silicones corresponding to formula (B) are chosen from the silicones of formula (E) below: 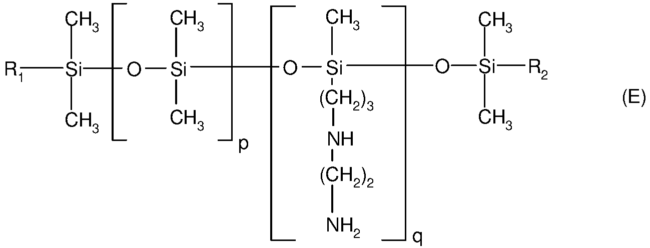 in which:
- p and q are numbers such that the sum (p + q) ranges from 1 to 1000, in particular from 50 to 350 and more particularly from 150 to 250; it being possible for p to denote a number from 0 to 999 and in particular from 49 to 349 and more particularly from 159 to 239, and for q to denote a number from 1 to 1000, in particular from 1 to 10 and more particularly from 1 to 5;
- R1 and R2, which are different, represent a hydroxyl or C₁-C₄ alkoxy radical, at least one of the radicals R1 or R2 denoting an alkoxy radical.

Preferably, the alkoxy radical is a methoxy radical.

The hydroxy/alkoxy mole ratio generally ranges from 1:0.8 to 1:1.1 and preferably from 1:0.9 to 1:1 and more particularly equals 1:0.95.

The weight-average molecular mass (Mw) of the silicone preferably ranges from 2000 to 200 000, even more particularly from 5000 to 100 000 and more particularly from 10 000 to 50 000.

The commercial products comprising silicones of structure (D) or (E) may include in their composition one or more other amino silicones the structure of which is different from formula (D) or (E).

A product containing amino silicones of structure (D) is sold by Wacker under the name Belsil^{®} ADM 652.

A product containing amino silicones of structure (E) is sold by Wacker under the name Fluid WR 1300^{®}.

When these amino silicones are used, one particularly advantageous embodiment consists in using them in the form of an oil-in-water emulsion. The oil-in-water emulsion may comprise one or more surfactants. The surfactants may be of any nature but are preferably cationic and/or non-ionic. The number-average size of the silicone particles in the emulsion generally ranges from 3 nm to 500 nanometres. Preferably, in particular as amino silicones of formula (E), use is made of microemulsions the mean particle size of which ranges from 5 nm to 60 nanometers (limits included) and more particularly from 10 nm to 50 nanometers (limits included). Thus, use may be made according to the invention of the amino silicone microemulsions of formula (E) sold under the names Finish CT 96 E^{®} or SLM 28020^{®} by Wacker.

According to a fourth embodiment, the amino silicones corresponding to formula (B) are chosen from the silicones of formula (F) below: 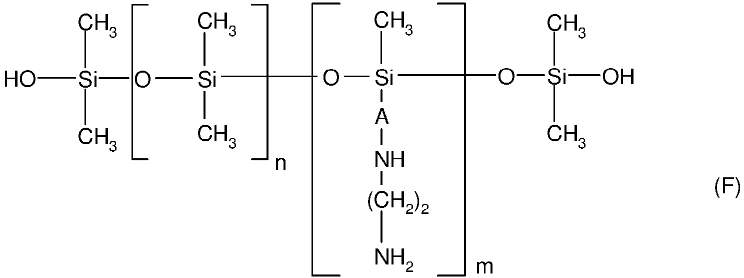 in which:
- m and n are numbers such that the sum (n + m) ranges from 1 to 2000 and in particular from 50 to 150, it being possible for n to denote a number from 0 to 1999 and especially from 49 to 149, and for m to denote a number from 1 to 2000 and especially from 1 to 10;
- A denotes a linear or branched alkylene radical having from 4 to 8 carbon atoms and preferably 4 carbon atoms. This radical is preferably linear.

The weight-average molecular mass (Mw) of these amino silicones preferably ranges from 2000 to 1 000 000 and even more particularly from 3500 to 200 000.

A silicone corresponding to this formula is, for example, the Xiameter MEM 8299 Emulsion from Dow Corning.

According to a fifth embodiment, the amino silicones corresponding to formula (B) are chosen from the silicones of formula (G) below: 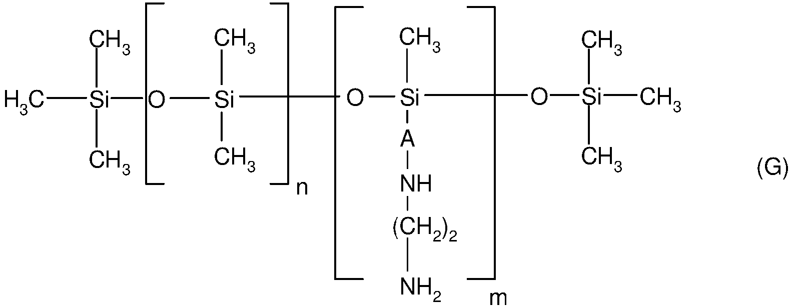 in which:
- m and n are numbers such that the sum (n + m) ranges from 1 to 2000 and in particular from 50 to 150, it being possible for n to denote a number from 0 to 1999 and especially from 49 to 149, and for m to denote a number from 1 to 2000 and especially from 1 to 10;
- A denotes a linear or branched alkylene radical having from 4 to 8 carbon atoms and preferably 4 carbon atoms. This radical is preferably branched.

The weight-average molecular mass (Mw) of these amino silicones preferably ranges from 500 to 1 000 000 and even more particularly from 1000 to 200 000.

A silicone corresponding to this formula is, for example, DC2-8566 Amino Fluid from Dow Corning;
c) the amino silicones corresponding to formula (H): 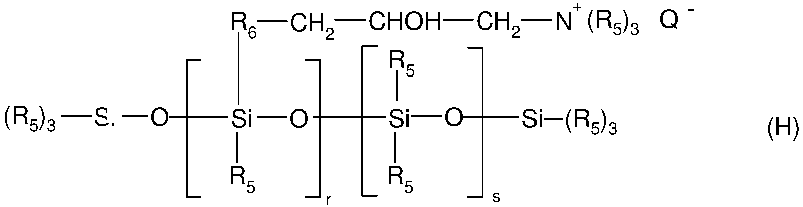 in which:
- R₅ represents a monovalent hydrocarbon-based radical having from 1 to 18 carbon atoms, and in particular a C₁-C₁₈ alkyl or C₂-C₁₈ alkenyl, for example methyl, radical;
- R₆ represents a divalent hydrocarbon-based radical, especially a C₁-C₁₈ alkylene radical or a divalent C₁-C₁₈, for example C₁-C₈, alkyleneoxy radical linked to the Si via an SiC bond;
- Q- is an anion such as a halide, especially chloride, ion or an organic acid salt, especially acetate;
- r represents a mean statistical value ranging from 2 to 20 and in particular from 2 to 8;
- s represents a mean statistical value ranging from 20 to 200 and in particular from 20 to 50.

Such amino silicones are especially described in patent US 4 185 087;
- d) the silicones comprising a quaternary ammonium having the formula below: 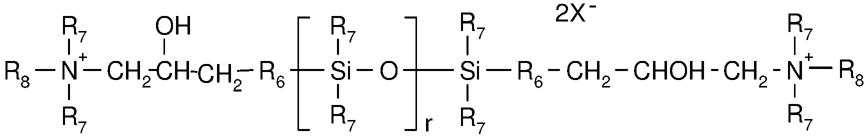 in which:
   - R₇, which may be identical or different, represent a monovalent hydrocarbon-based radical containing from 1 to 18 carbon atoms, and in particular a C₁-C₁₈ alkyl radical, a C₂-C₁₈ alkenyl radical or a ring comprising 5 or 6 carbon atoms, for example methyl;
   - R₆ represents a divalent hydrocarbon-based radical, especially a C₁-C₁₈ alkylene radical or a divalent C₁-C₁₈, for example C₁-C₈, alkyleneoxy radical linked to the Si via an SiC bond;
   - R₈, which may be identical or different, represent a hydrogen atom, a monovalent hydrocarbon-based radical having from 1 to 18 carbon atoms, and in particular a C₁-C₁₈ alkyl radical, a C₂-C₁₈ alkenyl radical or a radical -R₆-NHCOR₇;
   - X⁻ is an anion such as a halide, especially chloride, ion or an organic acid salt, especially acetate;
   - r represents a mean statistical value ranging from 2 to 200 and in particular from 5 to 100.

These silicones are described, for example, in patent application EP-A 0 530 974;
e) the amino silicones of formula (J): 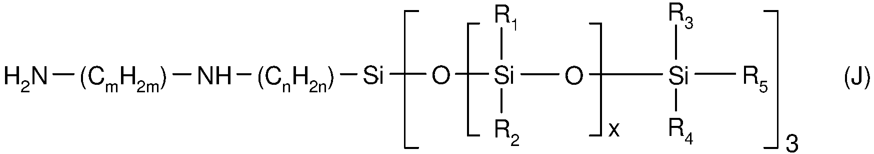 in which:
   - R₁, R₂, R₃ and R₄, which may be identical or different, denote a C₁-C₄ alkyl radical or a phenyl group,
   - R₅ denotes a C₁-C₄ alkyl radical or a hydroxyl group,
   - n is an integer ranging from 1 to 5,
   - m is an integer ranging from 1 to 5, and
   - x is chosen such that the amine number ranges from 0.01 to 1 meq/g;
f) the multiblock polyoxyalkylenated amino silicones, of the type (AB)n, A being a polysiloxane block and B being a polyoxyalkylenated block comprising at least one amine group.

Said silicones are preferably constituted of repeating units of the following general formulae:

[-(SiMe₂O)ₓSiMe₂ - R -N(R")- R'-O(C₂H₄O)ₐ(C₃H₆O)_{b} -R'-N(H)-R-]

or alternatively

[-(SiMe₂O)ₓSiMe₂ - R -N(R")- R' - O(C₂H₄O)ₐ(C₃H₆O)_{b} -]

in which:
- a is an integer greater than or equal to 1, preferably ranging from 5 to 200 and more particularly ranging from 10 to 100;
- b is an integer between 0 and 200, preferably ranging from 4 to 100 and more particularly between 5 and 30;
- x is an integer ranging from 1 to 10 000 and more particularly from 10 to 5000;
- R" is a hydrogen atom or a methyl;
- R, which may be identical or different, represent a divalent linear or branched C₂-C₁₂ hydrocarbon-based radical, optionally including one or more heteroatoms such as oxygen; preferably, R denotes an ethylene radical, a linear or branched propylene radical, a linear or branched butylene radical, or a - CH₂CH₂CH₂OCH₂CH(OH)CH₂- radical; preferentially R denotes a - CH₂CH₂CH₂OCH₂CH(OH)CH₂- radical;
- R', which may be identical or different, represent a linear or branched C₂-C₁₂ divalent hydrocarbon-based radical, optionally comprising one or more heteroatoms such as oxygen; preferably, R' denotes an ethylene radical, a linear or branched propylene radical, a linear or branched butylene radical, or a - CH₂CH₂CH₂OCH₂CH(OH)CH₂- radical; preferentially, R' denotes -CH(CH₃)-CH₂-.

The siloxane blocks preferably represent 50 mol% to 95 mol% of the total weight of the silicone, more particularly from 70 mol% to 85 mol%.

The amine content is preferably between 0.02 and 0.5 meq/g of copolymer in a 30% solution in dipropylene glycol, more particularly between 0.05 and 0.2.

The weight-average molecular mass (Mw) of the silicone is preferably between 5000 and 1 000 000 and more particularly between 10 000 and 200 000.

Mention may be made especially of the silicones sold under the names Silsoft A-843 or Silsoft A+ by Momentive;
g) and mixtures thereof.

Preferably, the silicone(s) are chosen from amino silicones.

Preferably, the liquid fatty substance(s) are chosen from liquid fatty esters different from the monoester(s) i); more preferentially from triglyceride oils of plant or synthetic origin, monoesters of monoacids and of monoalcohols, and mixtures thereof; even more preferentially from liquid triglycerides of fatty acids comprising from 6 to 30 carbon atoms, liquid esters of saturated or unsaturated, linear or branched C₁-C₂₆ aliphatic monoacids and of saturated or unsaturated, linear or branched C₁-C₂₆ aliphatic monoalcohols, the total number of carbon atoms of the liquid esters being greater than or equal to 10, and mixtures thereof; even better still from jojoba oil, ethyl palmitate, ethyl laurate, ethyl myristate, ethyl octanoate, ethyl caprate, and mixtures thereof.

According to one preferred embodiment of the invention, the liquid fatty substance(s) are chosen from triglyceride oils of plant or synthetic origin; more preferentially from liquid triglycerides of fatty acids comprising from 6 to 30 carbon atoms, sunflower oil, maize oil, soya bean oil, marrow oil, grapeseed oil, sesame oil, hazelnut oil, apricot oil, macadamia oil, arara oil, castor oil, avocado oil, olive oil, rapeseed oil, copra oil, wheatgerm oil, sweet almond oil, safflower oil, candlenut oil, camelina oil, tamanu oil, babassu oil and pracaxi oil, caprylic/capric acid triglycerides, jojoba oil, shea butter oil, and mixtures thereof; even more preferentially, the composition comprises jojoba oil.

According to another preferred embodiment of the invention, the liquid fatty substance(s) are chosen from monoesters of monoacids and of monoalcohols; more preferentially from ethyl palmitate, isopropyl palmitate, alkyl myristates such as isopropyl or ethyl myristate, isocetyl stearate, ethyl laurate, 2-ethylhexyl isononanoate, isononyl isononanoate, ethyl octanoate, ethyl caprate, isodecyl neopentanoate, isostearyl neopentanoate, and mixtures thereof; even more preferentially from ethyl palmitate, ethyl laurate, ethyl myristate, ethyl octanoate, ethyl caprate, and mixtures thereof.

Preferably, the total content of liquid fatty substance(s) present in the composition according to the invention is between 1% and 70% by weight, more preferentially between 10% and 65% by weight, even more preferentially between 20% and 60% by weight, better still between 30% and 55% by weight, relative to the total weight of the composition.

### Polyols

The composition according to the present invention comprises one or more polyols including at least one diol.

For the purposes of the present invention, the term "polyol" is intended to mean an organic compound constituted of a hydrocarbon-based chain optionally interrupted with one or more oxygen atoms and bearing at least two free hydroxyl groups (-OH) borne by different carbon atoms, this compound possibly being cyclic or acyclic, linear or branched, saturated or unsaturated, and in the liquid state at ambient temperature (25°C) and atmospheric pressure.

Preferably, the polyol(s) according to the invention are non-cyclic and non-aromatic.

For the purposes of the present invention, the term "diol" is intended to mean an organic compound constituted of a hydrocarbon-based chain optionally interrupted with one or more oxygen atoms and bearing only two free hydroxyl groups (-OH) borne by different carbon atoms, this compound possibly being cyclic or acyclic, linear or branched, saturated or unsaturated, and in the liquid state at ambient temperature (25°C) and atmospheric pressure (i.e. 1.013×10⁵ Pa).

The polyols, including the diols, according to the invention preferably comprise in their structure a number of carbon atoms of less than 8, for example ranging from 2 to 8, more preferentially ranging from 2 to 6, better still from 2 to 5. The polyols according to the invention are different from the fatty alcohols previously described.

More particularly, the polyol(s) that may be used according to the invention comprise from 2 to 10 hydroxyl groups, more preferentially from 2 to 5 hydroxyl groups and even more preferentially from 2 to 3 hydroxyl groups.

Preferably, said polyol(s) that may be used according to the invention are chosen from polyols comprising at least three carbon atoms and ethylene glycol, and are preferably chosen from propylene glycol, 1,3-propanediol, 1,3-butylene glycol, 1,2-pentanediol, dipropylene glycol, hexylene glycol, pentylene glycol, glycerol and ethylene glycol, and a mixture of these compounds.

According to one preferred embodiment of the invention, the composition comprises one or more polyols comprising 2 hydroxyl groups, that is to say one or more diols; more preferentially, the diol(s) are chosen from propylene glycol, 1,3-propanediol, 1,3-butylene glycol, 1,2-pentanediol, dipropylene glycol, hexylene glycol, pentylene glycol, ethylene glycol, and mixtures thereof; even more preferentially the composition comprises 1,3-propanediol.

Preferably, the total content of polyol(s) present in the composition according to the invention is between 5% and 70% by weight, more preferentially between 10% and 65% by weight, even more preferentially between 15% and 60% by weight, relative to the total weight of the composition.

Preferably, the total content of diol(s) present in the composition according to the invention is between 0.5% and 50% by weight, more preferentially between 1% and 30% by weight, even more preferentially between 2% and 10% by weight, relative to the total weight of the composition.

The weight ratio of, on the one hand, the total content of monoester(s) of a fatty acid and of (poly)glycerol to, on the other hand, the total content of diol(s) present in the composition according to the invention is greater than or equal to 1.5; preferably greater than or equal to 2, more preferentially greater than or equal to 2.5, better still greater than 3.

Preferably, this ratio is within the range extending from 1.5 to 15, more preferentially from 2 to 10, even more preferentially from 3 to 7, better still from 3.5 to 5.5.

Preferably, the weight ratio of, on the one hand, the total content of monoester(s) of a fatty acid and of (poly)glycerol to, on the other hand, the total content of polyol(s) present in the composition according to the invention ranges from 0.1 to 20, preferentially from 0.5 to 10, better still from 0.7 to 5, even better still from 0.8 to 3.

### Cationic surfactants

The composition according to the invention comprises one or more cationic agents chosen from cationic polymers and/or cationic surfactants.

The cationic surfactants are advantageously chosen from optionally polyoxyalkylenated primary, secondary or tertiary fatty amine salts; quaternary ammonium salts, and mixtures thereof.

As quaternary ammonium salts, mention may be made especially of:
- the quaternary ammonium salts of formula (Ia): 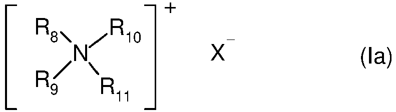 in which:
   the groups R₈ to R₁₁, which may be identical or different, represent a linear or branched aliphatic group containing from 1 to 30 carbon atoms, or an aromatic group such as aryl or alkylaryl, at least one of the groups R₈ to R₁₁ containing from 8 to 30 and preferably from 12 to 24 carbon atoms; it is possible for the aliphatic groups to comprise heteroatoms such as, in particular, oxygen, nitrogen, sulfur and halogens; and
- X⁻ is an anion chosen especially from the group of halides, phosphates, acetates, lactates, (C₁-C₄)alkyl sulfates, (C₁-C₄)alkylsulfonates or (C₁-C₄)alkylarylsulfonates;

The aliphatic groups R₈ to R₁₁ may be chosen from C₁-C₃₀ alkyl, C₁-C₃₀ alkoxy, (C₂-C₆) polyoxyalkylene, C₁-C₃₀ alkylamide, (C₁₂-C₂₂)alkylamido(C₂-C₆)alkyl, (C₁₂-C₂₂)alkyl acetate, and C₁-C₃₀ hydroxyalkyl groups.

Mention may be made especially of tetraalkylammonium halides, especially chlorides, such as dialkyldimethylammonium or alkyltrimethylammonium chlorides in which the alkyl group comprises from 12 to 22 carbon atoms, in particular behenyltrimethylammonium chloride, distearyldimethylammonium chloride, cetyltrimethylammonium chloride and benzyldimethylstearylammonium chloride.

Mention may also be made of palmitylamidopropyltrimethylammonium or stearamidopropyldimethyl-(myristyl acetate)-ammonium halides, and especially chlorides; especially the product sold under the name Ceraphyl^{®} 70 by the company Van Dyk.
- the quaternary ammonium salts of imidazoline of formula (IIa): 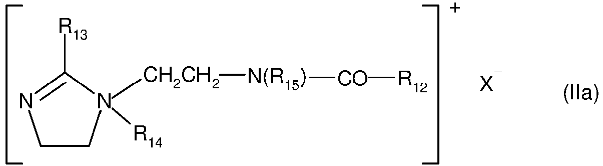 in which
   R₁₂ represents an alkenyl or alkyl group comprising from 8 to 30 carbon atoms, for example derived from tallow fatty acids,
   R₁₃ represents a hydrogen atom, a C₁-C₄ alkyl group or an alkenyl or alkyl group comprising from 8 to 30 carbon atoms,
   R₁₄ represents a C₁-C₄ alkyl group,
   R₁₅ represents a hydrogen atom or a C₁-C₄ alkyl group,
   X⁻ is an anion chosen especially from the group of the halides, phosphates, acetates, lactates, (C₁-C₄)alkyl sulfates, and (C₁-C₄)alkylsulfonates or (C₁-C₄)alkylarylsulfonates;
   Preferably, R₁₂ and R₁₃ denote a mixture of alkenyl or alkyl groups comprising from 12 to 21 carbon atoms, for example derived from tallow fatty acids, R₁₄ denotes a methyl group and R₁₅ denotes a hydrogen atom. Such a product is sold, for example, under the name Rewoquat^{®} W75 or W90 by the company Evonik.
- the quaternary di- or triammonium salts of formula (IIIa): 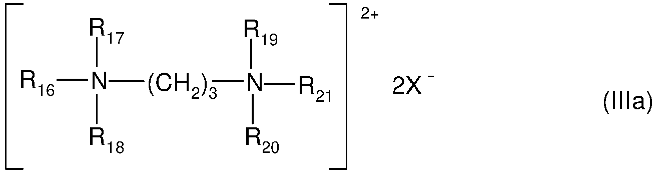 in which:
   - R₁₆ denotes an alkyl group comprising from 16 to 30 carbon atoms, which is optionally hydroxylated and/or optionally interrupted with one or more oxygen atoms,
   - R₁₇ denotes hydrogen, an alkyl group comprising from 1 to 4 carbon atoms or a group -(CH₂)₃-N⁺(R₁₆ₐ)(R₁₇ₐ)(R₁₈ₐ), R₁₆ₐ, R₁₇ₐ and R₁₈ₐ, which may be identical or different, denoting hydrogen or an alkyl group comprising from 1 to 4 carbon atoms,
   - R₁₈, R₁₉, R₂₀ and R₂₁, which may be identical or different, denote hydrogen or an alkyl group comprising from 1 to 4 carbon atoms, and
   - X⁻ is an anion, chosen especially from the group of the halides, acetates, phosphates, nitrates, (C₁-C₄)alkyl sulfates, (C₁-C₄)alkylsulfonates and (C₁-C₄)alkylarylsulfonates, in particular methyl sulfate and ethyl sulfate.

Such compounds are, for example, Finquat CT-P (Quaternium 89) and Finquat CT (Quaternium 75), sold by the company Finetex;
- quaternary ammonium salts containing one or more ester functions, of formula (IVa) below: 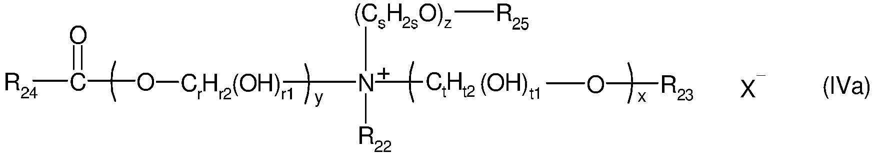 in which:
   - R₂₂ is chosen from C₁-C₆ alkyl groups and C₁-C₆ hydroxyalkyl or dihydroxyalkyl groups,
   - R₂₃ is chosen from the group R₂₆-C(=O)-; linear or branched, saturated or unsaturated C₁-C₂₂ hydrocarbon-based groups R₂₇; and a hydrogen atom,
   - R₂₅ is chosen from the group R₂₈-C(=O)-; linear or branched, saturated or unsaturated C₁-C₆ hydrocarbon-based groups R₂₉; and a hydrogen atom,
   - R₂₄, R₂₆ and R₂₈, which may be identical or different, are chosen from linear or branched, saturated or unsaturated C₇-C₂₁ hydrocarbon-based groups,
   - r, s and t, which may be identical or different, are integers ranging from 2 to 6,
   - r1 and t1, which may be identical or different, are equal to 0 or 1,
   - y is an integer ranging from 1 to 10,
   - x and z, which may be identical or different, are integers ranging from 0 to 10,
   - X⁻ is an anion,

it being understood that r2 + r1 = 2r and t1 + t2 = 2t, and that the sum x + y + z ranges from 1 to 15,
with the proviso that when x = 0 then R₂₃ denotes R₂₇ and that when z = 0 then R₂₅ denotes R₂₉.

The alkyl groups R₂₂ may be linear or branched, preferably linear. Preferably, R₂₂ denotes a methyl, ethyl, hydroxyethyl or dihydroxypropyl group, and more particularly a methyl or ethyl group.

Advantageously, the sum x + y + z is from 1 to 10.

When R₂₃ is a hydrocarbon-based group R₂₇, it may comprise from 12 to 22 carbon atoms, or else may comprise from 1 to 3 carbon atoms.

When R₂₅ is a hydrocarbon-based group R₂₉, it preferably contains 1 to 3 carbon atoms.

Advantageously, R₂₄, R₂₆ and R₂₈, which may be identical or different, are chosen from linear or branched, saturated or unsaturated C₁₁-C₂₁ hydrocarbon-based groups, and more particularly from linear or branched C₁₁-C₂₁ alkyl and alkenyl groups.

Preferably, x and z, which may be identical or different, are equal to 0 or 1.

Advantageously, y is equal to 1.

Preferably, r, s and t, which may be identical or different, are equal to 2 or 3, and even more particularly are equal to 2.

The anion X⁻ is preferably a halide, preferably chloride, bromide or iodide, a (C₁-C₄)alkyl sulfate, a (C₁-C₄)alkylsulfonate or a (C₁-C₄)alkylarylsulfonate, a methanesulfonate, a phosphate, a nitrate, a tosylate, an anion derived from organic acid such as an acetate or a lactate or any other anion that is compatible with the ammonium bearing an ester function. The anion X⁻ is more particularly a chloride, a methyl sulfate or an ethyl sulfate.

Use is more particularly made, in the composition according to the invention, of the ammonium salts of formula (IVa) in which:
- R₂₂ denotes a methyl or ethyl group,
- x and y are equal to 1,
- z is equal to 0 or 1,
- r, s and t are equal to 2,
- R₂₃ is chosen from the group R₂₆-C(=O)-; methyl, ethyl or C₁₄-C₂₂ hydrocarbon-based groups, and a hydrogen atom,
- R₂₅ is chosen from the group R₂₈-C(=O)-; and a hydrogen atom,
- R₂₄, R₂₆ and R₂₈, which may be identical or different, are chosen from linear or branched, saturated or unsaturated C₁₃-C₁₇ hydrocarbon-based groups, and preferably from linear or branched, saturated or unsaturated C₁₃-C₁₇ alkyl and alkenyl groups.

Advantageously, the hydrocarbon-based groups are linear.

Among the compounds of formula (IVa), mention may be made of the salts, especially the chloride or methyl sulfate of diacyloxyethyldimethylammonium, diacyloxyethylhydroxyethylmethylammonium, monoacyloxyethyldihydroxy-ethylmethylammonium, triacyloxyethylmethylammonium or monoacyloxyethyl-hydroxyethyldimethylammonium, and mixtures thereof. The acyl groups preferably contain 14 to 18 carbon atoms and are derived more particularly from a plant oil such as palm oil or sunflower oil. When the compound contains several acyl groups, these groups may be identical or different.

These products are obtained, for example, by direct esterification of triethanolamine, triisopropanolamine, alkyldiethanolamine or alkyldiisopropanolamine, which are optionally oxyalkylenated, with fatty acids or with fatty acid mixtures especially of plant or animal origin, or by transesterification of the methyl esters thereof. This esterification may be followed by a quaternization by means of an alkylating agent such as an alkyl halide, preferably methyl or ethyl halide, a dialkyl sulfate, preferably dimethyl or diethyl sulfate, methyl methanesulfonate, methyl para-toluenesulfonate, glycol chlorohydrin or glycerol chlorohydrin. Such compounds are sold, for example, under the names Dehyquart^{®} by the company Henkel, Stepanquat^{®} by the company Stepan, Noxamium^{®} by the company CECA or Rewoquat^{®} WE 18 by the company Evonik.

The composition according to the invention may contain, for example, a mixture of quaternary ammonium monoester, diester and triester salts with a weight majority of diester salts. Use may also be made of the ammonium salts containing at least one ester function that are described in patents US-A-4 874 554 and US-A-4 137 180. Use may also be made of behenoylhydroxypropyltrimethylammonium chloride, for example, sold by the company Kao under the name Quartamin BTC 131.

Preferably, the ammonium salts containing at least one ester function contain two ester functions.

Preferably, the cationic surfactants are chosen from those of formula (Ia) or (IVa).

According to one preferred embodiment of the invention, the composition comprises one or more cationic surfactants; more preferentially, the cationic surfactant(s) are chosen from cetyltrimethylammonium, behenyltrimethylammonium and dipalmitoylethylhydroxyethylmethylammonium salts and mixtures thereof; even more preferentially from behenyltrimethylammonium chloride or methosulfate, cetyltrimethylammonium chloride or methosulfate, dipalmitoylethylhydroxyethylmethylammonium chloride or methosulfate, and mixtures thereof.

Preferably, when the composition according to the invention comprises one or more cationic surfactants, the total content of cationic surfactant(s) present in the composition according to the invention is between 0.05% and 5% by weight, more preferentially between 0.1% and 3% by weight, even more preferentially between 0.2% and 3% by weight, relative to the total weight of the composition.

Preferably, the total content of surfactant(s) present in the composition according to the invention is between 5% and 45% by weight, more preferentially between 15% and 35% by weight, even more preferentially between 20% and 35% by weight, relative to the total weight of the composition.

### Cationic polymers

The composition according to the invention comprises one or more cationic agents chosen from cationic polymers and/or cationic surfactants.

For the purposes of the present invention, the expression "cationic polymer" denotes any non-silicone (not comprising any silicon atoms) polymer containing cationic groups and/or groups that can be ionized into cationic groups and not containing any anionic groups and/or groups that can be ionized into anionic groups.

The cationic polymers that may be used preferably have a weight-average molar mass (Mw) of between 500 and 5×10⁶ approximately and preferably between 10³ and 3×10⁶ approximately.

Among the cationic polymers, mention may be made more preferentially of:
(1) homopolymers or copolymers derived from acrylic or methacrylic esters or amides and comprising at least one of the units of following formulae: 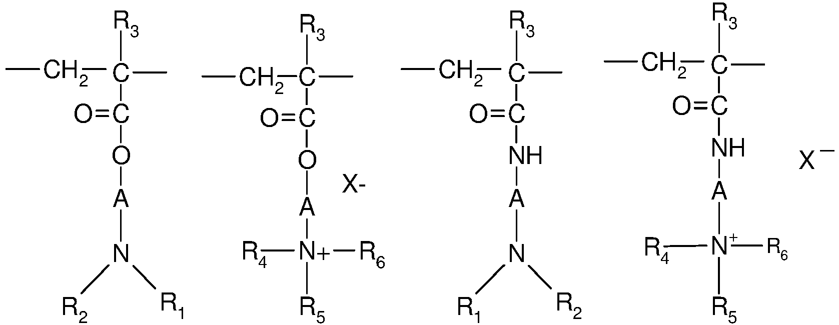 in which:
   - R₃, which may be identical or different, denote a hydrogen atom or a CH₃ radical;
   - A, which may be identical or different, represent a linear or branched divalent alkyl group of 1 to 6 carbon atoms, preferably 2 or 3 carbon atoms, or a hydroxyalkyl group of 1 to 4 carbon atoms;
   - R₄, R₅ and R₆, which may be identical or different, represent an alkyl group containing from 1 to 18 carbon atoms or a benzyl radical; preferably an alkyl group containing from 1 to 6 carbon atoms;
   - R₁ and R₂, which may be identical or different, represent a hydrogen atom or an alkyl group containing from 1 to 6 carbon atoms, and preferably methyl or ethyl;
   - X denotes an anion derived from a mineral or organic acid, such as a methosulfate anion or a halide such as chloride or bromide.

The copolymers of family (1) may also contain one or more units deriving from comonomers that may be chosen from the family of the acrylamides, methacrylamides, diacetone acrylamides, acrylamides and methacrylamides substituted on the nitrogen with lower (C₁-C₄) alkyls, acrylic or methacrylic acid esters, vinyllactams such as vinylpyrrolidone or vinylcaprolactam, and vinyl esters.

Among these copolymers of family (1), mention may be made of:
- copolymers of acrylamide and of dimethylaminoethyl methacrylate quaternized with dimethyl sulfate or with a dimethyl halide, such as that sold under the name Hercofloc by the company Hercules,
- copolymers of acrylamide and of methacryloyloxyethyltrimethylammonium chloride, such as the products sold under the name Bina Quat P 100 by the company Ciba Geigy,
- the copolymer of acrylamide and of methacryloyloxyethyltrimethylammonium methosulfate, such as that sold under the name Reten by the company Hercules,
- quaternized or non-quaternized vinylpyrrolidone/dialkylaminoalkyl acrylate or methacrylate copolymers, such as the products sold under the name Gafquat by the company ISP, for instance Gafquat 734 or Gafquat 755, or alternatively the products known as Copolymer 845, 958 and 937. These polymers are described in detail in French patents 2 077 143 and 2 393 573;
- dimethylaminoethyl methacrylate/vinylcaprolactam/vinylpyrrolidone terpolymers, such as the product sold under the name Gaffix VC 713 by the company ISP,
- vinylpyrrolidone/methacrylamidopropyldimethylamine copolymers, such as the copolymers sold under the name Styleze CC 10 by ISP;
- quaternized vinylpyrrolidone/dimethylaminopropylmethacrylamide copolymers such as the product sold under the name Gafquat HS 100 by the company ISP,
- polymers, preferably crosslinked polymers, of methacryloyloxy(C₁-C₄)alkyl tri(C₁-C₄)alkylammonium salts, such as the polymers obtained by homopolymerization of dimethylaminoethyl methacrylate quaternized with methyl chloride, or by copolymerization of acrylamide with dimethylaminoethyl methacrylate quaternized with methyl chloride, the homo- or copolymerization being followed by crosslinking with an olefinically unsaturated compound, in particular methylenebisacrylamide. Use may be made more particularly of a crosslinked acrylamide/methacryloyloxyethyltrimethylammonium chloride copolymer (20/80 by weight) in the form of a dispersion comprising 50% by weight of said copolymer in mineral oil. This dispersion is sold under the name Salcare^{®} SC 92 by the company Ciba. Use may also be made of a crosslinked methacryloyloxyethyltrimethylammonium chloride homopolymer comprising approximately 50% by weight of the homopolymer in mineral oil or in a liquid ester. These dispersions are sold under the names Salcare^{®} SC 95 and Salcare^{®} SC 96 by the company Ciba.

(2) cationic polysaccharides, in particular cationic celluloses and galactomannan gums. Among the cationic polysaccharides, mention may be made more particularly of cellulose ether derivatives comprising quaternary ammonium groups, cationic cellulose copolymers or cellulose derivatives grafted with a water-soluble quaternary ammonium monomer and cationic galactomannan gums.

The cellulose ether derivatives comprising quaternary ammonium groups are especially described in patent FR 1 492 597, and mention may be made of the polymers sold under the name Ucare Polymer JR (JR 400 LT, JR 125 and JR 30M) or LR (LR 400 and LR 30M) by the company Amerchol, and also the polyquaternium-67 in particular sold under the name SoftCAT by the company Dow Chemical. These polymers are also defined in the CTFA dictionary as quaternary ammoniums of hydroxyethylcellulose that have reacted with an epoxide substituted with a trimethylammonium group.

Cationic cellulose copolymers or cellulose derivatives grafted with a water-soluble quaternary ammonium monomer are described in particular in patent US 4 131 576, and mention may be made of hydroxyalkyl celluloses, for instance hydroxymethyl, hydroxyethyl or hydroxypropyl celluloses grafted, in particular, with a methacryloylethyltrimethylammonium, methacrylamidopropyltrimethylammonium or dimethyldiallylammonium salt. The copolymers corresponding to this definition are more particularly polyquaternium-4 and polyquaternium-10, in particular sold under the name Celquat L 200, Celquat H 100, Celquat SC230 and Celquat SC240 by the company National Starch.

The cationic galactomannan gums are described more particularly in patents US 3 589 578 and US 4 031 307, and mention may be made of guar gums comprising cationic trialkylammonium groups. Use is made, for example, of guar gums modified with a 2,3-epoxypropyltrimethylammonium salt (for example, a chloride). Such products are in particular sold under the names Jaguar C13 S, Jaguar C 15, Jaguar C 17 and Jaguar C162 by the company Rhodia.

(3) polymers constituted of piperazinyl units and of divalent alkylene or hydroxyalkylene radicals containing linear or branched chains, optionally interrupted with oxygen, sulfur or nitrogen atoms or with aromatic or heterocyclic rings, and also the oxidation and/or quaternization products of these polymers.

(4) water-soluble polyaminoamides prepared in particular by polycondensation of an acidic compound with a polyamine; these polyaminoamides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyaminoamide; these polyaminoamides can be alkylated or, if they comprise one or more tertiary amine functions, they can be quaternized.

(5) polyaminoamide derivatives resulting from the condensation of polyalkylene polyamines with polycarboxylic acids followed by alkylation with difunctional agents. Mention may be made, for example, of adipic acid/dialkylaminohydroxyalkyldialkylenetriamine polymers in which the alkyl radical comprises from 1 to 4 carbon atoms and preferably denotes methyl, ethyl or propyl. Among these derivatives, mention may be made more particularly of the adipic acid/dimethylaminohydroxypropyl/diethylenetriamine polymers sold under the name Cartaretine F, F4 or F8 by the company Sandoz.

(6) polymers obtained by reacting a polyalkylene polyamine comprising two primary amine groups and at least one secondary amine group with a dicarboxylic acid chosen from diglycolic acid and saturated aliphatic dicarboxylic acids containing from 3 to 8 carbon atoms; the mole ratio between the polyalkylene polyamine and the dicarboxylic acid preferably being between 0.8:1 and 1.4:1; the resulting polyaminoamide being reacted with epichlorohydrin in a mole ratio of epichlorohydrin relative to the secondary amine group of the polyaminoamide preferably of between 0.5:1 and 1.8:1. Polymers of this type are sold in particular under the name Hercosett 57 by the company Hercules Inc. or else under the name PD 170 or Delsette 101 by the company Hercules in the case of the adipic acid/epoxypropyl/diethylenetriamine copolymer.

(7) cyclopolymers of alkyldiallylamine or of dialkyldiallylammonium, such as homopolymers or copolymers comprising, as main constituent of the chain, units corresponding to formula (XXXI) or (XXXII): 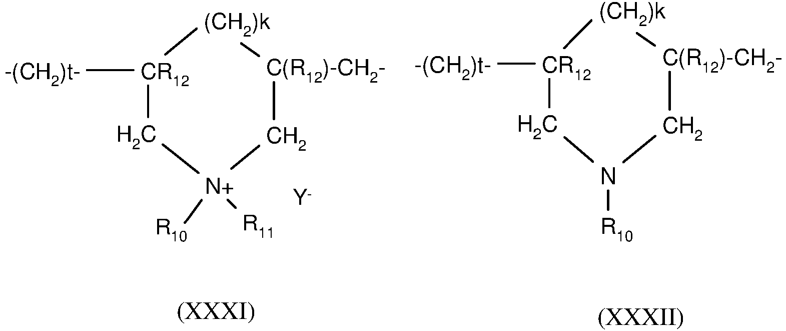 in which
- k and t are equal to 0 or 1, the sum k + t being equal to 1;
- R₁₂ denotes a hydrogen atom or a methyl radical;
- R₁₀ and R₁₁, independently of one another, denote a C₁-C₆ alkyl group, a C₁-C₅ hydroxyalkyl group, a C₁-C₄ amidoalkyl group; or alternatively R₁₀ and R₁₁ may denote, together with the nitrogen atom to which they are attached, a heterocyclic group such as piperidinyl or morpholinyl; R₁₀ and R₁₁, independently of one another, preferably denote a C₁-C₄ alkyl group;
- Y⁻ is an anion such as bromide, chloride, acetate, borate, citrate, tartrate, bisulfate, bisulfite, sulfate or phosphate.

Mention may be made more particularly of the homopolymer of dimethyldiallylammonium salts (for example chloride) for example sold under the name Merquat 100 by the company Nalco and the copolymers of diallyldimethylammonium salts (for example chloride) and of acrylamide, sold in particular under the name Merquat 550 or Merquat 7SPR.

(8) quaternary diammonium polymers comprising repeating units of formula (XXXIII) below: 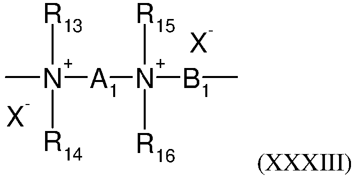 in which:
- R₁₃, R₁₄, R₁₅ and R₁₆, which may be identical or different, represent aliphatic, alicyclic or arylaliphatic radicals comprising from 1 to 20 carbon atoms or C₁-C₁₂ hydroxyalkyl aliphatic radicals;
   or else R₁₃, R₁₄, R₁₅ and R₁₆, together or separately, form, with the nitrogen atoms to which they are attached, heterocycles optionally comprising a second non-nitrogen heteroatom;
   or else R₁₃, R₁₄, R₁₅ and R₁₆ represent a linear or branched C₁-C₆ alkyl radical substituted with a nitrile, ester, acyl, amide or -CO-O-R₁₇-D or -CO-NH-R₁₇-D group, where R₁₇ is an alkylene and D is a quaternary ammonium group;
- A₁ and B₁ represent divalent polymethylene groups comprising from 2 to 20 carbon atoms which may be linear or branched, and saturated or unsaturated, and which may contain, linked to or inserted in the main chain, one or more aromatic rings, or one or more oxygen or sulfur atoms or sulfoxide, sulfone, disulfide, amino, alkylamino, hydroxyl, quaternary ammonium, ureido, amide or ester groups; and
- X⁻ denotes an anion derived from a mineral or organic acid;
   it being understood that A₁, R₁₃ and R₁₅ can form, with the two nitrogen atoms to which they are attached, a piperazine ring;
   in addition, if A₁ denotes a linear or branched, saturated or unsaturated alkylene or hydroxyalkylene radical, B₁ may also denote a group (CH₂)ₙ-CO-D-OC-(CH₂)ₚ- with n and p, which may be identical or different, being integers ranging from 2 to 20, and D denoting:
      a) a glycol residue of formula -O-Z-O-, in which Z denotes a linear or branched hydrocarbon-based radical or a group corresponding to one of the following formulae: -(CH₂CH₂O)ₓ-CH₂CH₂- and -[CH₂CH(CH₃)O]_{y}-CH₂CH(CH₃)-, where x and y denote an integer from 1 to 4, representing a defined and unique degree of polymerization or any number from 1 to 4 representing an average degree of polymerization;
      b) a bis-secondary diamine residue, such as a piperazine derivative;
      c) a bis-primary diamine residue of formula -NH-Y-NH-, in which Y denotes a linear or branched hydrocarbon-based radical, or else the divalent radical -CH₂-CH₂-S-S-CH₂-CH₂-;
      d) an ureylene group of formula -NH-CO-NH-.

Preferably, X⁻ is an anion, such as chloride or bromide. These polymers have a number-average molar mass (Mn) generally of between 1000 and 100 000.

Mention may be made more particularly of polymers that are constituted of repeating units corresponding to the formula: 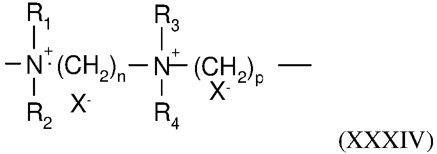 in which R₁, R₂, R₃ and R₄, which may be identical or different, denote an alkyl or hydroxyalkyl radical containing from 1 to 4 carbon atoms, n and p are integers ranging from 2 to 20, and X⁻ is an anion derived from a mineral or organic acid.

A compound constituted by units of formula (XXXIV) that is particularly preferred is the one for which R₁, R₂, R₃ and R₄ represent a methyl radical and n = 3, p = 6 and X = Cl, which is known as Hexadimethrine chloride according to the INCI (CTFA) nomenclature.

(9) polyquaternary ammonium polymers comprising units of formula (XXXV): 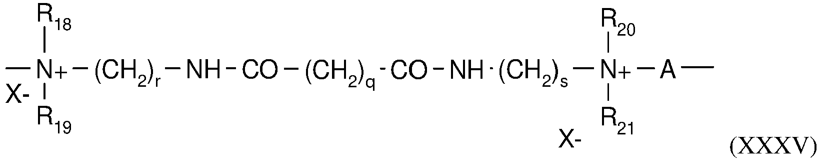 in which:
- R₁₈, R₁₉, R₂₀ and R₂₁, which may be identical or different, represent a hydrogen atom or a methyl, ethyl, propyl, β-hydroxyethyl, β-hydroxypropyl or -CH₂CH₂(OCH₂CH₂)ₚOH radical, where p is equal to 0 or to an integer of between 1 and 6, with the proviso that R₁₈, R₁₉, R₂₀ and R₂₁ do not simultaneously represent a hydrogen atom,
- r and s, which may be identical or different, are integers between 1 and 6,
- q is equal to 0 or to an integer between 1 and 34,
- X⁻ denotes an anion, such as a halide,
- A denotes a divalent dihalide radical or preferably represents -CH₂-CH₂-O-CH₂-CH₂-.

Examples that may be mentioned include the products Mirapol^{®} A 15, Mirapol^{®} AD1, Mirapol^{®} AZ1 and Mirapol^{®} 175 sold by the company Miranol.

(10) quaternary polymers of vinylpyrrolidone and of vinylimidazole, for instance the products sold under the names Luviquat^{®} FC 905, FC 550 and FC 370 by the company BASF.

(11) polyamines such as Polyquart^{®} H sold by Cognis, referred to under the name Polyethylene glycol (15) tallow polyamine in the CTFA dictionary.

(12) polymers comprising in their structure:
(a) one or more units corresponding to formula (A) below: 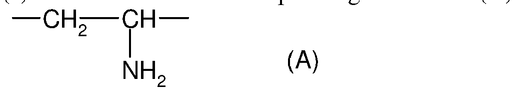
(b) optionally one or more units corresponding to formula (B) below:

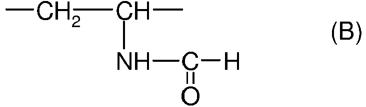

In other words, these polymers may especially be chosen from homopolymers or copolymers comprising one or more units derived from vinylamine and optionally one or more units derived from vinylformamide.

Preferably, these cationic polymers are chosen from polymers comprising, in their structure, from 5 mol% to 100 mol% of units corresponding to the formula (A) and from 0 to 95 mol% of units corresponding to the formula (B), preferentially from 10 mol% to 100 mol% of units corresponding to the formula (A) and from 0% to 90 mol% of units corresponding to the formula (B).

These polymers may be obtained, for example, by partial hydrolysis of polyvinylformamide. This hydrolysis may take place in acidic or basic medium.

The weight-average molecular weight of said polymer, measured by light scattering, may range from 1000 to 3 000 000 g/mol, preferably from 10 000 to 1 000 000 and more particularly from 100 000 to 500 000 g/mol.

The polymers comprising units of formula (A) and optionally units of formula (B) are sold in particular under the name Lupamin by the company BASF, for instance, in a non-limiting way, the products provided under the names Lupamin 9095, Lupamin 5095, Lupamin 1095, Lupamin 9030 (or Luviquat 9030) and Lupamin 9010.

### (13) and mixtures thereof.

Other cationic polymers that may be used in the context of the invention are cationic proteins or cationic protein hydrolyzates, polyalkyleneimines, in particular polyethyleneimines, polymers comprising vinylpyridine or vinylpyridinium units, condensates of polyamines and of epichlorohydrin, quaternary polyureylenes and chitin derivatives.

Preferably, the cationic polymer(s) are chosen from those of families (1), (2), (7) and (8) described above; more preferentially from those of family (2) described above.

Among the cationic polymers mentioned above, the ones that may preferably be used are cationic polysaccharides, in particular cationic celluloses and cationic galactomannan gums, and in particular quaternary cellulose ether derivatives such as the products sold under the name JR 400 by the company Amerchol and under the name SoftCAT by the company Dow Chemical, cationic cyclopolymers, in particular dimethyldiallylammonium salt (for example chloride) homopolymers or copolymers, sold under the names Merquat 100, Merquat 550 and Merquat S by the company Nalco, optionally crosslinked homopolymers or copolymers of methacryloyloxy(C₁-C₄)alkyltri(C₁-C₄)alkylammonium salts; polymers constituted of repeating units corresponding to formula (XXXIV) above, in particular the compound Hexadimethrine chloride according to the INCI (CTFA) nomenclature, and mixtures thereof.

According to one preferred embodiment of the invention, the composition comprises one or more cationic polymers; more preferentially chosen from cationic polysaccharides; even more preferentially from derivatives of cellulose ethers comprising quaternary ammonium groups; even better still the composition comprises polyquaternium-67.

Preferably, when the composition comprises one or more cationic polymers, the total content of cationic polymer(s) in the composition according to the invention is between 0.01% and 3% by weight, more preferentially between 0.05% and 2% by weight, even more preferentially between 0.1% and 1% by weight, relative to the total weight of the composition.

Advantageously, the cationic agent(s), chosen from cationic polymers and/or cationic surfactants, is(are) non-silicone cationic agents.

### Water

The composition according to the present invention comprises water.

Preferably, the total water content ranges from 2% to 20% by weight, and even better still from 2% to 10% by weight, relative to the total weight of the composition.

According to one preferred embodiment of the invention, the weight ratio of, on the one hand, the total water content to, on the other hand, the total content of liquid fatty substance(s) is included in the range extending from 0.01 to 3; preferably from 0.05 to 2.5; more preferentially from 0.075 to 2; even more preferentially from 0.1 to 1.6.

Preferably, the pH of the aqueous phase is between 3 and 8, more preferentially between 3.5 and 7, even more preferentially between 4.5 and 5.5.

The pH may be adjusted to the desired value by means of basifying agents or acidifying agents that are customarily used.

The composition according to the invention may also contain additives normally used in cosmetics, such as thickeners, preservatives, fragrances and colourants.

These additives may be present in the composition according to the invention in an amount ranging from 0% to 20% by weight relative to the total weight of the composition.

Those skilled in the art will take care to select these optional additives and the amounts thereof so that they do not harm the properties of the compositions of the present invention.

The composition according to the invention may be in the form of a shampoo or of a conditioner.

The composition may be used on wet or dry hair, in rinse-out or leave-in mode.

A subject of the invention is also a process for washing and/or for caring for human keratin fibres such as the hair, comprising at least one step of applying, to said human keratin fibres, a composition as described above.

According to a preferred embodiment of the invention, a step of rinsing the human keratin fibres is performed after the step(s) of applying a composition according to the invention to said fibres.

A step of drying the human keratin fibres may be envisaged in the process according to the invention, especially using a heating means such as a hair dryer, a straightening iron, a steam iron or a heating hood; the heating means possibly heating to a temperature ranging from 150°C to 230°C, preferably from 200°C to 220°C.

A subject of the invention is also the use of the composition according to the invention for washing and/or caring for human keratin fibres such as the hair.

The examples that follow serve to illustrate the invention without, however, being limiting in nature.

### EXAMPLES:

### Example 1:

The cosmetic compositions (A) and (B) according to the invention are prepared from the ingredients shown in the table below, the amounts of which are expressed as weight percentages of active material (AM).

| | **Composition A** | **Composition B** |
|---|---|---|
| Polyglyceryl-4 laurate | 16.5 | 16.42 |
| Polyglyceryl-5 oleate | 5.5 | 5.47 |
| 1,3-Propanediol | 5 | 5.02 |
| Glycerol | 15 | 15.05 |
| Isopropyl myristate | 24.7 | - |
| Isopropyl palmitate | 24.7 | 49.5 |
| Polyquaternium-67 | 0.3 | 0.3 |
| Water | qs 100 | qs 100 |
| Weight ratio of the total content of monoester(s) of a fatty acid and of (poly)glycerol to the total content of diol(s) | 4.4 | 4.3 |
| Weight ratio of the total content of monoester(s) of a fatty acid and of (poly)glycerol to the total content of polyol(s) | 1.1 | 1.1 |

### Evaluation of the detergent power

The detergent power or compositions (A) and (B) according to the invention was then evaluated, in comparison with that of a commercial washing oil.

Locks of 2.7 g of natural caucasian hair were used for these evaluations.

Protocol: 37 mg of artificial sebum are applied to a lock of hair. The lock is then left to stand for one hour.

1 g of one of compositions A or B to be evaluated is then applied to a lock of hair of 2.7 g.

Finally, each lock is rinsed and left to dry in the open air.

The protocol is repeated 2 successive times per lock of hair, each lock of hair being treated with just one of the compositions to be evaluated.

The visual appearance of the 2 locks respectively treated with compositions (A) and (B) is then evaluated with the naked eye in comparison to a lock of 2.7 g of natural caucasian hair washed a first time with water and then a second time with a commercial shampoo of DOP chamomile type.

The visual appearance is evaluated by three experts who assign a score ranging from 0 to 5.
- 0 / 5 corresponding to a lock of hair with a very very dirty appearance,
- 1 / 5 corresponding to a lock of hair with a very dirty appearance,
- 2 / 5 corresponding to a lock of hair with a dirty appearance,
- 3 / 5 corresponding to a lock of hair with a not very clean appearance,
- 4 / 5 corresponding to a lock of hair with a clean appearance, and
- 5 / 5 corresponding to a lock of hair with a very clean appearance.

The results are collated in the table below.

| Compositions | Detergent power after 3 cycles of the protocol |
|---|---|
| Composition A | 4/5 |
| Composition B | 4/5 |
| Commercial washing oil* | 3/5 |
| DOP shampoo | 5/5 |

| | |
|---|---|
| * ENJOIL shampoo oil from Fauvert | |

The locks of hair treated with composition (A) or composition (B) according to the invention appear cleaner than the lock of hair treated with the commercial comparative washing oil.

### Hair care evaluation

A lock of 2.7 g of sensitized caucasian hair (SA 20) is used for this evaluation.

Composition A according to the invention is applied to a first lock of hair according to a bath ratio of 0.4 g of composition / gram of hair.

The comparative composition* (*commercial washing oil above) is applied to a second lock of hair according to a bath ratio of 0.4 g of composition / gram of hair.

A sensory evaluation of the disentangling of the wet hair, of the smooth nature to the touch (smooth feel) and of the suppleness is then carried out for each of the locks by 3 experts.

Each cosmetic attribute is evaluated by means of a score between 0 and 5:
- 0 / 5 corresponding to a lock of hair of very very unsatisfactory appearance,
- 1 / 5 corresponding to a lock of hair of very unsatisfactory appearance,
- 2 / 5 corresponding to a lock of hair of unsatisfactory appearance,
- 3 / 5 corresponding to a lock of hair of not very satisfactory appearance,
- 4 / 5 corresponding to a lock of hair of satisfactory appearance, and
- 5 / 5 corresponding to a lock of hair of very satisfactory appearance.

The results are collated in the table below (mean of the three scores from the 3 experts).

| | Composition A | Comparative composition |
|---|---|---|
| Disentangling (wet hair) | 5 / 5 | 3 / 5 |
| Smooth feel | 4 / 5 | 2 / 5 |
| Suppleness | 4 / 5 | 2 / 5 |

The lock of hair treated with the composition according to the invention has a level of conditioning which is greater than the lock of hair treated with the comparative composition, in particular in terms of disentangling, of smooth feel and of suppleness of the hair.

### Example 2:

The cosmetic compositions (A1) and (A2) according to the invention and the comparative composition (B) are prepared from the ingredients shown in the table below, the amounts of which are expressed as weight percentages of active material (AM).

| | **Composition A1** | **Composition A2** | **Composition B** |
|---|---|---|---|
| Polyquaternium-67 | 0,3 | - | - |
| Brassicamidopropyl dimethylamine + salicylic acid in a weight ratio 3:1 | - | 4 | - |
| Glycerin | 15 | 15 | 15 |
| Propanediol | 5 | 5 | 5 |
| Polyglyceryl-4 laurate | 16.5 | 16.5 | 16.5 |
| Polyglyceryl-5 oleate | 5.5 | 5.5 | 5.5 |
| Perfume | 0.5 | 0.5 | 0.5 |
| Isopropyl myristate | 24.7 | 22.85 | 24.7 |
| Isopropyl palmitate | 24.7 | 22.85 | 24.7 |
| Water | qs 100 | qs 100 | qs 100 |
| Weight ratio of the total content of monoester(s) of a fatty acid and of (poly)glycerol to the total content of diol(s) | 4.4 | 4.4 | 4.4 |
| Weight ratio of the total content of monoester(s) of a fatty acid and of (poly)glycerol to the total content of polvol(s) | 1.1 | 1.1 | 1.1 |

### Protocol:

The compositions A1, A2 and B are applied onto weak sensitized locks (alkaline solubility=20%, SA20), previously moistened, in a standard manner by kneading the locks for 15 seconds (6 passages between the fingers from top to ends) at a rate of 1 g composition per 2.7 g hair. After 5 minutes break, the hair is rinsed with water at 35°C (300 L/h flow) for 10s (15 passes between the fingers).

The hair is then wrung, dried in an oven and disentangled.

### Evaluation 1:

In order to evaluate the ease of use of the 3 compositions A1, A2 and B, 5 experts evaluated in a blind test, the ease with which the compositions are spread over the entire lock (ease spreading) according to the protocol above. Then, the experts rated each composition on a scale from 0 (very difficult) to 5 (very very easy).

Each composition is evaluated by means of a score between 0 and 5:
- 0 / 5 corresponding to a composition very difficult to spread over the lock,
- 1 / 5 corresponding to a composition difficult to spread over the lock,
- 2 / 5 corresponding to a composition not easy to spread over the lock,
- 3 / 5 corresponding to a composition easy to spread over the lock,
- 4 / 5 corresponding to a composition very easy to spread over the lock, and
- 5 / 5 corresponding to a composition very very easy to spread over the lock.

The results are collated in the table below (mean of the five scores from the 5 experts).

| | Ease of use |
|---|---|
| Composition A1 | 4.1 ± 0.5 / 5 |
| Composition A2 | 3.3 ± 0.4 / 5 |
| Comparative composition B | 2.2 ± 0.4 / 5 |

Thus, the compositions A1 and A2 according to the invention are easier to spread on the hair than the comparative composition B.

### Evaluation 2:

The presence of static electricity for the locks of hair treated according to the above protocol was then evaluated.

Thus, each lock quickly pass 5 times between the fingers of the expert. We then observe whether the hair is lifting in all directions (fly away effect) or if they remain disciplined.

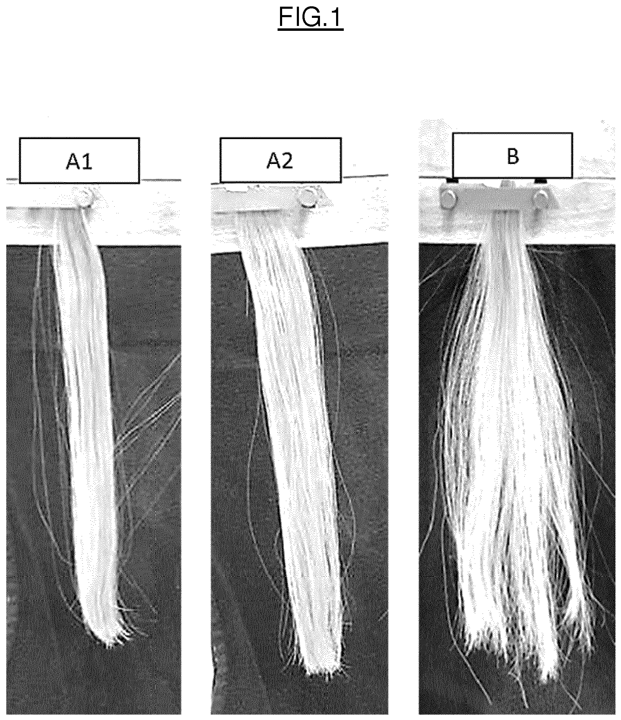

The results are shown in Figure 1.
Figure 1 shows:
- on the left, the lock of hair treated with the composition A1,
- in the middle, the lock of hair treated with the composition A2,
- on the right, the lock of hair treated with the composition B.

We can observe that the locks of hair respectively treated with the composition A1 and A2 according to the invention, stay perfectly disciplined.

In parallel, the lock of hair treated with the comparative composition B lifts in different directions (fly away effect).

Thus, the locks of hair treated with compositions A1 or A2 according to the invention present less static electricity than the lock of hair treated with the comparative composition B.

## Claims

1. Cosmetic composition comprising:
i) one or more monoesters of a fatty acid and of (poly)glycerol in a total content of greater than 10% by weight relative to the total weight of the composition,
ii) one or more fatty substances, liquid at 30°C and at 1.013×10⁵ Pa, chosen from hydrocarbons, fatty alcohols comprising from 8 to 40 carbon atoms, fatty esters comprising from 8 to 40 carbon atoms, fatty ethers comprising from 8 to 40 carbon atoms, silicones and mixtures thereof,
iii) one or more polyols including at least one diol, and
iv) one or more cationic agents chosen from cationic polymers and/or cationic surfactants;
v) water, in a content ranging from 1% to 20% by weight relative to the total weight of the composition;
it being understood that the weight ratio of, on the one hand, the total content of monoester(s) of a fatty acid and of (poly)glycerol to, on the other hand, the total content of diol(s) is greater than or equal to 1.5.

2. Composition according to the preceding claim, **characterized in that** the weight ratio of, on the one hand, the total content of monoester(s) of a fatty acid and of (poly)glycerol to, on the other hand, the total content of diol(s) is greater than or equal to 2, preferably greater than or equal to 2.5, more preferentially greater than or equal to 3.

3. Composition according to either one of the preceding claims, **characterized in that** the monoester(s) of a fatty acid and of (poly)glyceryl comprise on average from 1 to 30 mol of glycerol, preferably from 2 to 10 mol of glycerol, more preferentially from 3 to 8 mol of glycerol.

4. Composition according to any one of the preceding claims, **characterized in that** the monoester(s) of a fatty acid and of (poly)glycerol are chosen from the compounds of formula (I) below: 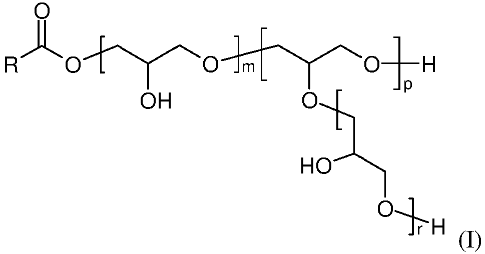 in which:
R represents a linear or branched, saturated or unsaturated C₇-C₃₉, preferably C₉-C₂₉, more preferentially C₁₁-C₂₁, hydrocarbon-based chain;
m, p and r represent an integer between 0 and 30, and are such that the sum of the integers m, p and r is between 1 and 30, better still between 2 and 10, and even better still between 3 and 8;
even more preferentially, p=r=0, and m is an integer between 1 and 30, better still m is an integer between 2 and 10, or even between 3 and 8.

5. Composition according to any one of the preceding claims, **characterized in that** the monoester(s) of a fatty acid and of (poly)glycerol are chosen from monoesters of C₈-C₃₀ fatty acids and of (poly)glycerol; preferably from monoesters of C₈-C₃₀ fatty acids and of polyglycerol; more preferentially from monoesters of oleic acid and of polyglycerol, monoesters of lauric acid and of polyglycerol, and mixtures thereof; even more preferentially from tetraglyceryl monooleate (INCI name: polyglyceryl-4 oleate), pentaglyceryl monooleate (INCI name: polyglyceryl-5 oleate), tetraglyceryl monolaurate (INCI name: polyglyceryl-4 laurate), and mixtures thereof.

6. Composition according to any one of the preceding claims, **characterized in that** the total content of monoester(s) of a fatty acid and of (poly)glyceryl is between 10% and 40% by weight, preferably between 12% and 35% by weight, more preferentially between 15% and 30%, even more preferentially between 20% and 30% by weight, relative to the total weight of the composition.

7. Composition according to any one of the preceding claims, **characterized in that** the liquid fatty substance(s) are chosen from liquid fatty esters different from the monoester(s) i); preferably from triglyceride oils of plant or synthetic origin, monoesters of monoacids and of monoalcohols, and mixtures thereof; more preferentially from liquid triglycerides of fatty acids comprising from 6 to 30 carbon atoms, liquid esters of saturated or unsaturated, linear or branched C₁-C₂₆ aliphatic monoacids and of saturated or unsaturated, linear or branched C₁-C₂₆ aliphatic monoalcohols, the total number of carbon atoms of the liquid esters being greater than or equal to 10, and mixtures thereof.

8. Composition according to any one of the preceding claims, **characterized in that** the total content of liquid fatty substance(s) is between 1% and 70% by weight, preferably between 10% and 65% by weight, more preferentially between 20% and 60% by weight and even more preferentially between 30% and 55% by weight, relative to the total weight of the composition.

9. Composition according to any one of the preceding claims, **characterized in that** the polyol(s) are chosen from polyols comprising 2 hydroxyl or diol groups; preferably from propylene glycol, 1,3-propanediol, 1,3-butylene glycol, 1,2-pentanediol, dipropylene glycol, hexylene glycol, pentylene glycol, ethylene glycol, and mixtures thereof; more preferentially the composition comprises 1,3-propanediol; even more preferentially the total content of polyol(s) is between 5% and 70% by weight, better between 10% and 65% by weight, and even better between 15% and 60% by weight, relative to the total weight of the composition.

10. Composition according to any one of the preceding claims, **characterized in that** the weight ratio of, on the one hand, the total content of monoester(s) of a fatty acid and of (poly)glycerol to, on the other hand, the total content of polyol(s) ranges from 0.1 to 20, preferably from 0.5 to 10, more preferentially from 0.7 to 5, even more preferentially from 0.8 to 3.

11. Composition according to any one of the preceding claims, **characterized in that** it comprises water in a content ranging from 1.5% to 20% by weight, even more particularly from 2% to 20% by weight, and even better still from 2% to 10% by weight, relative to the total weight of the composition.

12. Composition according to the preceding claim, **characterized in that** the weight ratio of, on the one hand, the total water content to, on the other hand, the total content of liquid fatty substance(s) is included in the range extending from 0.01 to 3; preferably from 0.05 to 2.5; more preferentially from 0.075 to 2; even more preferentially from 0.1 to 1.6.

13. Composition according to any one of the preceding claims, **characterized in that** it comprises one or more cationic surfactants;
preferably chosen from cetyltrimethylammonium, behenyltrimethylammonium and dipalmitoylethylhydroxyethylmethylammonium salts, and mixtures thereof;
more preferentially in a total content of between 0.05% and 5% by weight, better still between 0.1% and 3% by weight, even better still between 0.2% and 3% by weight, relative to the total weight of the composition.

14. Composition according to any one of the preceding claims, **characterized in that** it comprises one or more cationic polymers;
preferably chosen from cationic polysaccharides; more preferentially from derivatives of cellulose ethers comprising quaternary ammonium groups;
even more preferentially in a total content of between 0.01% and 3% by weight, better still between 0.05% and 2% by weight, even better still between 0.1% and 1% by weight, relative to the total weight of the composition.

15. Process for washing and/or caring for human keratin fibres such as the hair, comprising at least one step of applying, to said human keratin fibres, a composition according to any one of Claims 1 to 14, optionally followed by a step of rinsing said human keratin fibres.

## Patentansprüche

1. Kosmetische Zusammensetzung umfassend:
i) einen oder mehrere Monoester von einer Fettsäure und (Poly)glycerol in einem Gesamtgehalt von mehr als 10 Gew.-%, bezogen auf das Gesamtgewicht der Zusammensetzung,
ii) einen oder mehrere Fettstoffe, flüssig bei 30 °C und 1,013x10⁵ Pa, ausgewählt aus Kohlenwasserstoffen, Fettalkoholen umfassend von 8 bis 40 Kohlenstoffatome, Fettsäureester umfassend von 8 bis 40 Kohlenstoffatome, Fettsäurether umfassend von 8 bis 40 Kohlenstoffatomen, Siliconen und Gemischen davon,
iii) ein oder mehrere Polyole, einschließlich wenigstens eines Diols, und
iv) ein oder mehrere kationische Mittel ausgewählt aus kationischen Polymeren und/oder kationischen Tensiden;
v) Wasser in einem Gehalt in dem Bereich von 1 bis 20 Gew.-%, bezogen auf das Gesamtgewicht der Zusammensetzung;
wobei es sich versteht, dass das Gewichtsverhältnis von einerseits dem Gesamtgehalt an Monoester(n) von einer Fettsäure und (Poly)glycerol zu andererseits dem Gesamtgehalt an Diol(en) größer als oder gleich 1,5 ist.

2. Zusammensetzung nach dem vorstehenden Anspruch, **dadurch gekennzeichnet, dass** das Gewichtsverhältnis von einerseits dem Gesamtgehalt an Monoester(n) von einer Fettsäure und (Poly)glycerol zu andererseits dem Gesamtgehalt an Diol(en) größer als oder gleich 2, vorzugsweise größer als oder gleich 2,5, bevorzugter größer als oder gleich 3 ist.

3. Zusammensetzung nach einem der vorstehenden Ansprüche, **dadurch gekennzeichnet, dass** der oder die Monoester von einer Fettsäure und (Poly)glyceryl im Mittel von 1 bis 30 mol Glycerol, vorzugsweise von 2 bis 10 mol Glycerol, bevorzugter von 3 bis 8 mol Glycerol, enthalten.

4. Zusammensetzung nach einem der vorstehenden Ansprüche, **dadurch gekennzeichnet, dass** der oder die Monoester von einer Fettsäure und (Poly)glycerol ausgewählt sind aus den nachstehenden Verbindungen der Formel (I): 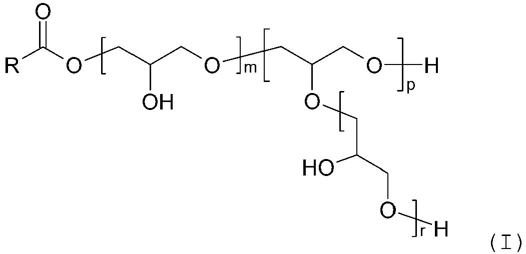 wobei:
R eine lineare oder verzweigte, gesättigte oder ungesättigte C₇-C₃₉-, vorzugsweise C₉-C₂₉-, bevorzugter C₁₁-C₂₁-Kette auf Kohlenwasserstoffbasis darstellt;
m, p und r eine ganze Zahl zwischen 0 und 30 darstellen und so sind, dass die Summe der ganzen Zahlen m, p und r zwischen 1 und 30, besser noch zwischen 2 und 10 und noch besser zwischen 3 und 8 beträgt;
noch bevorzugter p=r=0 und m eine ganze Zahl zwischen 1 und 30 ist, besser noch m eine ganze Zahl zwischen 2 und 10 oder sogar zwischen 3 und 8 ist.

5. Zusammensetzung nach einem der vorstehenden Ansprüche, **dadurch gekennzeichnet, dass** der oder die Monoester von einer Fettsäure und (Poly)glycerol ausgewählt sind aus Monoestern von C₈-C₃₀-Fettsäuren und (Poly)glycerol; vorzugsweise aus Monoestern von C₈-C₃₀-Fettsäuren und Polyglycerol; bevorzugter aus Monoestern von Ölsäure und Polyglycerol, Monoestern von Laurinsäure und Polyglycerol, und Gemischen davon; noch bevorzugter aus Tetraglycerylmonooleat (INCI-Name: Polyglyceryl-4-oleat), Pentaglycerylmonooleat (INCI-Name: Polyglyceryl-5-oleat), Tetraglycerylmonolaurat (INCI-Name: Polyglyceryl-4-laurat) und Gemischen davon.

6. Zusammensetzung nach einem der vorstehenden Ansprüche, **dadurch gekennzeichnet, dass** der Gesamtgehalt an Monoestern(n) von einer Fettsäure und (Poly)glyceryl zwischen 10 Gew.-% und 40 Gew.-%, vorzugsweise zwischen 12 Gew.-% und 35 Gew.-%, bevorzugter zwischen 15 Gew.-% und 30 Gew.-% und noch bevorzugter zwischen 20 Gew.-% und 30 Gew.-%, bezogen auf das Gesamtgewicht der Zusammensetzung, beträgt.

7. Zusammensetzung nach einem der vorstehenden Ansprüche, **dadurch gekennzeichnet, dass** die flüssige(n) Fettstoff(e) ausgewählt sind aus flüssigen Fettsäureestern, die von den Monoester(n) i) verschieden sind; vorzugsweise aus Triglyceridölen pflanzlichen oder synthetischen Ursprungs, Monoestern von Monosäuren und Monoalkoholen und Gemischen davon; bevorzugter aus flüssigen Triglyceriden von Fettsäuren mit 6 bis 30 Kohlenstoffatomen, flüssigen Estern gesättigter oder ungesättigter, linearer oder verzweigter aliphatischer C₁-C₂₆-Monosäuren und gesättigter oder ungesättigter, linearer oder verzweigter aliphatischer C₁-C₂₆-Monoalkohole, wobei die Gesamtzahl der Kohlenstoffatome der flüssigen Ester größer als oder gleich 10 ist, und Gemischen davon.

8. Zusammensetzung nach einem der vorstehenden Ansprüche, **dadurch gekennzeichnet, dass** der Gesamtgehalt an flüssige(n) Fettstoff(en) zwischen 1 Gew.-% und 70 Gew.-%, vorzugsweise zwischen 10 Gew.-% und 65 Gew.-%, bevorzugter zwischen 20 Gew.-% und 60 Gew.-% und noch bevorzugter zwischen 30 Gew.-% und 55 Gew.-%, bezogen auf das Gesamtgewicht der Zusammensetzung, beträgt.

9. Zusammensetzung nach einem der vorstehenden Ansprüche, **dadurch gekennzeichnet, dass** die Polyol(e) ausgewählt sind aus Polyolen umfassend 2 Hydroxy- oder Diolgruppen; vorzugsweise aus Propylenglycol, 1,3-Propandiol, 1,3-Butylenglycol, 1,2-Pentandiol, Dipropylenglycol, Hexylenglycol, Pentylenglycol, Ethylenglycol und Gemischen davon; wobei die Zusammensetzung bevorzugter 1,3-Propandiol umfasst; der Gesamtgehalt an Polyol(en) noch bevorzugter zwischen 5 Gew.-% und 70 Gew.-%, besser zwischen 10 Gew.-% und 65 Gew.-% und noch besser zwischen 15 Gew.-% und 60 Gew.-%, bezogen auf das Gesamtgewicht der Zusammensetzung, beträgt.

10. Zusammensetzung nach einem der vorstehenden Ansprüche, **dadurch gekennzeichnet, dass** das Gewichtsverhältnis zwischen einerseits dem Gesamtgehalt von Monoester(n) von einer Fettsäure und Polyglycerol und andererseits dem Gesamtgehalt an Polyol(en) in dem Bereich von 0,1 bis 20, vorzugsweise von 0,5 bis 10, bevorzugter von 0,7 bis 5 und noch bevorzugter von 0,8 bis 3 liegt.

11. Zusammensetzung nach einem der vorstehenden Ansprüche, **dadurch gekennzeichnet, dass** sie Wasser in einem Gehalt in dem Bereich von 1,5 Gew.-% bis 20 Gew.-%, insbesondere von 2 Gew.-% bis 20 Gew.-% und sogar noch besser von 2 Gew.-% bis 10 Gew.-%, bezogen auf das Gesamtgewicht der Zusammensetzung, umfasst.

12. Zusammensetzung nach dem vorstehenden Anspruch, **dadurch gekennzeichnet, dass** das Gewichtsverhältnis von einerseits dem Gesamtwassergehalt zu andererseits dem Gesamtgehalt an flüssiger(n) Fettstoff(en) in dem Bereich von 0,01 bis 3; vorzugsweise von 0,05 bis 2,5; bevorzugter von 0,075 bis 2; noch bevorzugter von 0,1 bis 1,6, liegt.

13. Zusammensetzung nach einem der vorstehenden Ansprüche, **dadurch gekennzeichnet, dass** sie ein oder mehrere kationische Tenside umfasst;
vorzugsweise ausgewählt aus Cetyltrimethylammonium-, Behenyltrimethylammonium- und Dipalmitoylethylhydroxyethylmethylammoniumsalzen und Gemischen davon;
bevorzugter in einem Gesamtgehalt von zwischen 0,05 Gew.-% und 5 Gew.-%, besser noch zwischen 0,1 Gew.-% und 3 Gew.-%, sogar noch besser zwischen 0,2 Gew.-% und 3 Gew.-%, bezogen auf das Gesamtgewicht der Zusammensetzung.

14. Zusammensetzung nach einem der vorstehenden Ansprüche, **dadurch gekennzeichnet, dass** sie ein oder mehrere kationische Polymere umfasst;
vorzugsweise ausgewählt aus kationischen Polysacchariden; bevorzugter aus Derivaten von Celluloseethern, die quartäre Ammoniumgruppen umfassen; noch bevorzugter in einem Gesamtgehalt von zwischen 0,01 Gew.-% und 3 Gew.-%, besser noch zwischen 0,05 Gew.-% und 2 Gew.-%, sogar noch besser zwischen 0,1 Gew.-% und 1 Gew.-%, bezogen auf das Gesamtgewicht der Zusammensetzung.

15. Verfahren zum Waschen und/oder Pflegen von menschlichen Keratinfasern, wie z.B. dem Haar, umfassend wenigstens einen Schritt des Aufbringens einer Zusammensetzung nach einem der Ansprüche 1 bis 14 auf die menschlichen Keratinfasern, gegebenenfalls gefolgt von einem Schritt des Spülens der menschlichen Keratinfasern.

## Revendications

1. Composition cosmétique comprenant :
i) un ou plusieurs monoesters d'acide gras et de (poly)glycérol en une teneur totale supérieure à 10% en poids par rapport au poids total de la composition,
ii) un ou plusieurs corps gras, liquides à 30°C et à 1,013.10⁵ Pa, choisis parmi les hydrocarbures, les alcools gras comprenant de 8 à 40 atomes de carbone, les esters gras comprenant de 8 à 40 atomes de carbone, les éthers gras comprenant de 8 à 40 atomes de carbone, les silicones et leurs mélanges.,
iii) un ou plusieurs polyols dont au moins un diol, et
iv) un ou plusieurs agents cationiques choisis parmi les polymères cationiques et/ou les tensioactifs cationiques ;
v) de l'eau, en une teneur allant de 1% à 20% en poids par rapport au poids total de la composition ;
étant entendu que le rapport pondéral de la teneur totale en monoester(s) d'acide gras et de (poly)glycérol d'une part sur la teneur totale en diol(s) d'autre part, est supérieur ou égal à 1,5.

2. Composition selon la revendication précédente, **caractérisée en ce que** le rapport pondéral de la teneur totale en monoester(s) d'acide gras et de (poly)glycérol d'une part sur la teneur totale en diol(s) d'autre part, est supérieur ou égal à 2, de préférence supérieur ou égal à 2,5, plus préférentiellement supérieur ou égal à 3.

3. Composition selon l'une quelconque des revendications précédentes, **caractérisée en ce que** le ou les monoesters d'acide gras et de (poly)glycérol comprennent en moyenne de 1 à 30 moles de glycérol ; de préférence de 2 à 10 moles de glycérol, plus préférentiellement de 3 à 8 moles de glycérol.

4. Composition selon l'une quelconque des revendications précédentes, **caractérisée en ce que** le ou les monoesters d'acide gras et de (poly)glycérol sont choisis parmi les composés de formule (I) suivante : 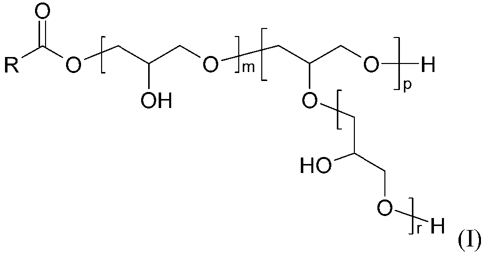 dans laquelle :
R représente une chaîne hydrocarbonée, linéaire ou ramifiée, saturée ou insaturée, en C₇-C₃₉, de préférence en C₉-C₂₉, plus préférentiellement en C₁₁-C₂₁ ;
m, p et r représentent un nombre entier compris entre 0 et 30, et sont tels que la somme des entiers m, p et r est comprise entre 1 et 30, mieux entre 2 et 10, et encore mieux entre 3 et 8 ;
plus préférentiellement encore, p=r=0, et m est un entier entre 1 et 30, mieux m est un entier entre 2 et 10, voire entre 3 et 8.

5. Composition selon l'une quelconque des revendications précédentes, **caractérisée en ce que** le ou les monoesters d'acide gras et de (poly)glycérol sont choisis parmi les monoesters d'acides gras en C₈-C₃₀ et de (poly)glycérol ; de préférence parmi les monoesters d'acides gras en C₈-C₃₀ et de polyglycérol ; plus préférentiellement parmi les monoesters d'acide oléique et de polyglycérol, les monoesters d'acide laurique et de polyglycérol, et leurs mélanges ; plus préférentiellement encore parmi le monooléate de tétraglycéryle (nom INCI : polyglyceryl-4 oleate), le monooléate de pentaglycéryle (nom INCI : polyglyceryl-5 oleate), le monolaurate de tétraglycéryle (nom INCI : polyglyceryl-4 laurate), et leurs mélanges.

6. Composition selon l'une quelconque des revendications précédentes, **caractérisée en ce que** la teneur totale en monoester(s) d'acide gras et de (poly)glycérol est comprise entre 10% et 40% en poids, de préférence entre 12% et 35% en poids, plus préférentiellement entre 15% et 30%, plus préférentiellement encore entre 20% et 30% en poids, par rapport au poids total de la composition.

7. Composition selon l'une quelconque des revendications précédentes, **caractérisée en ce que** le ou les corps gras liquides sont choisis parmi les esters gras liquides différents du ou des monoesters i) ; de préférence parmi les huiles triglycérides d'origine végétale ou synthétique, les monoesters de monoacides et de monoalcools, et leurs mélanges ; plus préférentiellement parmi les triglycérides liquides d'acides gras comportant de 6 à 30 atomes de carbone, les esters liquides de monoacides aliphatiques saturés ou insaturés, linéaires ou ramifiés, en C₁-C₂₆ et de monoalcool aliphatiques saturés ou insaturés, linéaires ou ramifiés, en C₁-C₂₆, le nombre total d'atomes de carbone des esters liquides étant supérieur ou égal à 10, et leurs mélanges.

8. Composition selon l'une quelconque des revendications précédentes, **caractérisée en ce que** la teneur totale en corps gras liquide(s) est comprise entre 1% et 70% en poids, de préférence entre 10% et 65% en poids, plus préférentiellement entre 20% et 60% en poids et plus préférentiellement encore entre 30% et 55% en poids, par rapport au poids total de la composition.

9. Composition selon l'une quelconque des revendications précédentes, **caractérisée en ce que** le ou les polyols sont choisis parmi les polyols comprenant 2 groupements hydroxy ou diols; plus préférentiellement parmi le propylène glycol, le 1,3-propanediol, le 1,3-butylène glycol, le pentane-1,2-diol, le dipropylène glycol, l'hexylène glycol, le pentylène glycol, l'éthylène glycol, et leurs mélanges ; plus préférentiellement encore la composition comprend le 1,3-propanediol ; encore plus préférentiellement la teneur totale en polyol(s) est entre 5% et 70% en poids, mieux entre 10% et 65% en poids, et encore mieux entre 15% et 60% en poids, par rapport au poids total de la composition.

10. Composition selon l'une quelconque des revendications précédentes, **caractérisée en ce que** le rapport pondéral de la teneur totale en monoester(s) d'acide gras et de (poly)glycérol d'une part sur la teneur totale en polyol(s) d'autre part, va de 0,1 à 20, de préférence de 0,5 à 10, plus préférentiellement de 0,7 à 5, plus préférentiellement encore de 0,8 à 3.

11. Composition selon l'une quelconque des revendications précédentes, **caractérisée en ce qu'**elle comprend de l'eau en une teneur allant de 1,5% à 20% en poids, encore plus particulièrement de 2% à 20% en poids, et encore mieux de 2% à 10% en poids, par rapport au poids total de la composition.

12. Composition selon la revendication précédente, **caractérisée en ce que** le rapport pondéral de la teneur totale en eau d'une part, sur la teneur totale en corps gras liquide(s) d'autre part, est compris dans la gamme allant de 0,01 à 3 ; de préférence de 0,05 à 2,5 ; plus préférentiellement de 0,075 à 2 ; plus préférentiellement encore de 0,1 à 1,6.

13. Composition selon l'une quelconque des revendications précédentes, **caractérisée en ce qu'**elle comprend un ou plusieurs tensioactifs cationiques ;
de préférence choisis parmi les sels de cétyltriméthylammonium, de béhényltriméthylammonium, de dipalmitoyléthylhydroxyéthylméthylammonium, et leurs mélanges ;
plus préférentiellement en une teneur totale comprise entre 0,05% et 5% en poids, mieux entre 0,1% et 3% en poids, encore mieux entre 0,2% et 3% en poids, par rapport au poids total de la composition.

14. Composition selon l'une quelconque des revendications précédentes, **caractérisée en ce qu'**elle comprend un ou plusieurs polymères cationiques ;
de préférence choisis parmi les polysaccharides cationiques ; plus préférentiellement parmi les dérivés d'éthers de cellulose comportant des groupements ammonium quaternaires ;
encore plus préférentiellement encore en une teneur totale comprise entre 0,01% et 3% en poids, mieux entre 0,05% et 2% en poids, mieux encore entre 0,1% et 1% en poids, par rapport au poids total de la composition.

15. Procédé de lavage et/ou de soin des fibres kératiniques humaines telles que les cheveux, comprenant au moins une étape d'application sur lesdites fibres kératiniques humaines, d'une composition selon l'une quelconque des revendications 1 à 14, éventuellement suivie d'une étape de rinçage desdites fibres kératiniques humaines.
